# EUROPÄISCHE PATENTSCHRIFT

(11) **EP 3 143 119 B1**
(45) Veröffentlichungstag und Bekanntmachung des Hinweises auf die Patenterteilung: **26.02.2020**
(21) Anmeldenummer: 15720320.9
(22) Anmeldetag: 29.04.2015
(51) Int. Cl.: C12Q 1/6806, C12N 13/00, C12N 15/10, G01N 1/40, B01L 3/00, C12N 1/06

(54) **VORRICHTUNG UND VERFAHREN ZUM AUFBEREITEN EINER BIOLOGISCHEN PROBE UND ANALYSESYSTEM ZUM ANALYSIEREN EINER BIOLOGISCHEN PROBE**
DEVICE AND METHOD TO PREPARE A BIOLOGICAL SAMPLE AND SYSTEM FOR ANALYSIS OF A BIOLOGICAL SAMPLE
PROCÉDÉ ET DISPOSITIF POUR TRAITER UN ÉCHANTILLON DE MATIÈRE BIOLOGIQUE ET SYSTEME D'ANALYSE D'UN ÉCHANTILLON DE MATIÈRE BIOLOGIQUE

(30) Priorität: 15.05.2014 DE 102014209188
(43) Veröffentlichungstag der Anmeldung: 22.03.2017
(73) Patentinhaber: Robert Bosch GmbH, 70442 Stuttgart (DE)
(72) Erfinder: BRETTSCHNEIDER, Thomas, 71229 Leonberg (DE); HOFFMANN, Jochen, 71229 Leonberg (DE); LEMUTH, Karin, 70195 Stuttgart (DE)
(86) Internationale Anmeldenummer: PCT/EP2015/059265
(87) Internationale Veröffentlichungsnummer: WO 2015/173016

(56) Entgegenhaltungen:
- WO-A1-2010/141131
- WO-A2-2008/072968
- WO-A2-2009/002580
- DE-A1-102011 086 235
- US-A1- 2011 129 931
- A. KLOKE ET AL: "The LabTube - a novel microfluidic platform for assay automation in laboratory centrifuges", LAB ON A CHIP, Bd. 14, Nr. 9, 3. Februar 2014 (2014-02-03), Seiten 1527-1537, XP055188025, ISSN: 1473-0197, DOI: 10.1039/c3lc51261d
- LANG NAN ET AL: "Emerging microfluidic devices for cell lysis: a review", LAB ON A CHIP, Bd. 14, Nr. 6, 4. Dezember 2013 (2013-12-04), Seite 1060, XP055188014, ISSN: 1473-0197, DOI: 10.1039/c3lc51133b

## Beschreibung

### Stand der Technik

Die vorliegende Erfindung bezieht sich auf eine Vorrichtung zum Aufbereiten einer biologischen Probe, auf ein entsprechendes Verfahren sowie ein Analysesystem zum Analysieren einer biologischen Probe.

Miniaturisierte mikrofluidische Diagnosesysteme, sogenannte Chiplabore bzw. LoCs (LoC; Lab-on-a-Chip) erlauben die miniaturisierte und integrierte Durchführung komplexer fluidischer Arbeitsabläufe für die Identifikation unterschiedlicher Pathogene. Viele Prozessschritte, die ansonsten üblicherweise von Hand in einem Labor durchgeführt werden, laufen automatisiert auf einem kompakten Einweg-Bauteil ab. In publizierten LoC-Systemen werden die zu untersuchenden Pathogene zunächst gefiltert und isoliert, dann aufgeschlossen bzw. lysiert und die DNA extrahiert. Anschließend werden bestimmte DNA-Abschnitte amplifiziert und identifiziert, beispielsweise im Rahmen einer Polymerase-Kettenreaktion bzw. PCR (englisch: Polymerase Chain Reaction). Diese Einzelprozesse werden in räumlich getrennten Bereichen auf dem LoC durchgeführt und nach jedem Prozessschritt automatisiert in den nächsten Bereich überführt. Hierzu werden üblicherweise Schichtaufbauten aus Polymeren oder Silizium verwendet.

Die Druckschrift WO 2010/141131 A1 offenbart einen Mehr-Proben-Chip für die Analyse von DNA.

Die Druckschrift WO 2008/072968 A2 offenbart ein Mikrofluidik-Gerätedesign, das Kapillaren aufweist.

Die Druckschrift US 2011/129931 A 1offenbart ein Mikrofluidik-System zur Erkennung von biologischen Elementen in einer Probe.

Die Druckschrift DE 10 2011 086235 A1 offenbart ein mikrofluidisches Filterelement zum Abscheiden von Probenbestandteilen aus einem biologischen Probenfluid.

Die Druckschrift WO 2009/002580 A2 offenbart ein Verfahren zur Lyse von Bakterien.

### Offenbarung der Erfindung

Vor diesem Hintergrund werden mit dem hier vorgestellten Ansatz eine Vorrichtung zum Aufbereiten einer biologischen Probe, weiterhin ein Verfahren, das unter Verwendung der Vorrichtung ausgeführt wird, sowie ein Analysesystem zum Analysieren einer biologischen Probe gemäß den Hauptansprüchen vorgestellt. Vorteilhafte Ausgestaltungen ergeben sich aus den jeweiligen Unteransprüchen und der nachfolgenden Beschreibung.

Mit einem Schichtaufbau, der eine Filterstruktur, die an zwei jeweils eine fluidische Schnittstelle aufweisende Teilkammern angrenzt, und bevorzugt auf der Filterstruktur ausgebildete metallische Strukturen aufweist, kann eine mikrofluidische Umgebung bzw. Struktur bereitgestellt werden, die es erlaubt, eine möglichst hohe Anzahl an Prozessschritten einer Aufbereitung organischer Zellen in einer einzelnen Kammer eines LoCs durchzuführen.

In einer Weiterbildung kann der hierin vorgeschlagene Schichtaufbau Bereiche aufweisen, die mit biochemischen Fängermolekülen für eine anschließende Detektion von Zielzellen ausgestattet sind. Dies ist insbesondere dann vorteilhaft, wenn der Schichtaufbau in eine mikrofluidische Umgebung mit einer flexiblen Membran integriert wird.

Gemäß dem hier vorgeschlagenen Ansatz können geeignete Prozessabläufe für Isolation, Lyse und ggf. DNA-Amplifikation von Zellen in einem einzigen Kompartiment durchgeführt werden. Neben einem sich daraus ergebenden geringeren Platzbedarf auf dem Chip kann der Diagnoseprozess erheblich vereinfacht werden, da es nicht länger erforderlich ist, für die jeweiligen Prozessabläufe lokal unterschiedliche Bedingungen auf dem Chip zu realisieren. Durch die Integration der Isolation von Zellen, Lyse und DNA-Amplifikation kann zudem vorteilhafterweise auf einen Schritt der DNA-Extraktion bzw. -Aufreinigung verzichtet werden, da PCR-inhibierende Substanzen wie z. B. Hämoglobin bereits bei der Filtration weggespült werden können. Dadurch kann eine Verkürzung der Prozesszeit, eine Verringerung und Vereinfachung von Prozessschritten und eine hohe Sensitivität bei der Identifikation von Zielzellen erreicht werden.

Bei einer gemäß dem hier vorgeschlagenen Ansatz durchgeführten Probenanalyse ohne räumliche Trennung zwischen einzelnen Prozessschritten kann ein Verlust des Analyten oder anderer Hilfsstoffe beim Transport der Flüssigkeiten durch Adsorption an Kanalwänden minimiert oder sogar ganz ausgeschaltet werden. Durch den Verzicht oder die Verringerung des Transports des Probefluids können zusätzlich Totvolumina und die Anzahl an Ventilen und Pumpen reduziert werden. Da die Pathogene in derselben Kammer aufgeschlossen werden, in der auch Abschnitte ihrer DNA vervielfältigt werden, kann eine höhere Sensitivität der Analyse erreicht werden.

Der hier vorgeschlagene Schichtaufbau aus Filter, Teilkammern und metallischen Strukturen ist besonders vorteilhaft mit Standardprozessen der Mikrosystemtechnik, insbesondere auf Basis von Siliziumwafern, herstellbar. Hierdurch können sehr kleine Strukturen wie die Filterporen massenfertigbar zu niedrigen Kosten hergestellt werden. Die Verwendung von Silizium ist zusätzlich aufgrund der hohen Wärmeleitfähigkeit vorteilhaft, da sich hiermit eine sehr homogene Temperaturverteilung einstellen lässt und beispielsweise eine schnelle Zyklierung der Temperatur im Rahmen einer PCR durchführen lässt, wodurch die gesamte Prozesszeit stark reduziert wird. Ferner kann durch den Einbau elektrisch leitfähiger Schichten auf externe Heizstrukturen verzichtet werden. Dies senkt die Fertigungskosten des Schichtsystems um den mit einer externen Versorgungseinheit einhergehenden Mehraufwand.

Zusätzlich lassen sich durch die elektrischen Strukturen effiziente Lyseverfahren einsetzen, beispielsweise eine Lyse mit elektrischen Feldern, die in der notwendigen Feldstärke nur mit viel Aufwand und sehr hohen Spannungen von extern zuführbar wären. Daneben erlaubt die hier vorgestellte Kombination eines Siliziumteils mit einer polymeren mikrofluidischen Umgebung die kostengünstige Verwendung von Einheitsfunktionen wie Ventilen und Pumpen auf Basis einer flexiblen Polymermembran.

Ein gemäß dem hier vorgeschlagenen Ansatz konzipiertes Analyseverfahren ermöglicht eine gezielte Amplifikation von Zielzellen-DNA, da ausschließlich Zielzellen auf der Filtermembran fixiert werden und damit in hoher Konzentration für die Vervielfältigung zur Verfügung stehen. Für den Gesamtablauf des Verfahrens werden weniger Puffer benötigt, da auf Puffer beispielsweise für die DNA-Extraktion, die enzymatische Lyse, etc. verzichtet werden kann. Das hierin vorgestellte Verfahren ermöglicht einen schnelleren Prozessablauf zusammen mit einer vereinfachten Prozessführung.

Es wird eine Vorrichtung zum Aufbereiten einer biologischen Probe vorgestellt, wobei die Vorrichtung die folgenden Merkmale aufweist:
ein erstes Substrat mit einer ersten Kavität, die einen ersten Kammerabschnitt einer Kammer zum Aufnehmen der biologischen Probe bildet;
ein zweites Substrat mit einer zweiten Kavität, die einen zweiten Kammerabschnitt der Kammer zum Aufnehmen der biologischen Probe bildet;
ein Filterelement mit einer Mehrzahl von Durchbrüchen, wobei das Filterelement zwischen der ersten Kavität und der zweiten Kavität ausgebildet ist, um bei einem Bewegen der biologischen Probe zwischen der ersten Kavität und der zweiten Kavität durch das Filterelement eine Mehrzahl von organischen Zellen der biologischen Probe auf der Mehrzahl von Durchbrüchen zurückzuhalten; und
eine elektrisch leitfähige Struktur, die an dem Filterelement angeordnet und ausgebildet ist, um eine Lyse der organischen Zellen herbeizuführen und/oder definierte Abschnitte der freigelegten DNA zu vervielfältigen und/oder nachzuweisen.

Die Vorrichtung kann Teil eines in einem medizinischen Labor eingesetzten Diagnosesystems zur Detektion von beispielsweise Bakterien, Krankheitserregern und Tumorzellen sein. Die Vorrichtung kann aber auch eigenständig zur Lyse organischer Zellen eingesetzt werden. Das erste und das zweite Substrat können zur Bildung der Kammer aus der ersten und der zweiten Kavität fluiddicht verbunden sein und somit im Verbund als ein Gehäuse für die Kammer verstanden werden. Das Filterelement kann in dem ersten Substrat oder alternativ in dem zweiten Substrat ausgebildet sein und wie auch die Kavitäten beispielsweise in einem geeigneten Ätzprozess entstanden sein. Zum Zurückhalten der organischen Zellen auf den Durchbrüchen des Filterelements kann ein lichtes Maß der Durchbrüche geringfügig geringer sein als ein Durchmesser der organischen Zellen aus der biologischen Probe, die für eine nachfolgende Diagnose interessant sind. Mit dem Zurückhalten der organischen Zellen auf den Durchbrüchen können die organischen Zellen von unerwünschten Bestandteilen der biologischen Probe wie beispielsweise Hämoglobin oder von nicht interessierenden weiteren Zellen getrennt und für die weitere Aufbereitung fixiert werden. Die elektrisch leitfähige Struktur kann so an dem Filterelement angeordnet sein, dass sie das Filterelement berührt und sich in einer Ebene parallel zu einer Hauptseite des Filterelements über einen Großteil der Hauptseite des Filterelements erstreckt.

Gemäß einer Ausführungsform der Vorrichtung können das erste Substrat und das zweite Substrat zumindest teilweise aus Silizium gebildet sein. Auf diese Weise kann die Vorrichtung sehr maßgenau mit geringen Toleranzen realisiert werden, wodurch die Funktionalität der Vorrichtung vorteilhaft gesteigert werden kann.

Ferner kann die Vorrichtung einen ersten Fluidkanal zwischen einer von der ersten Kavität abgewandten Hauptseite des ersten Substrats und der ersten Kavität und einen zweiten Fluidkanal zwischen der Hauptseite des ersten Substrats und der zweiten Kavität aufweisen. Der erste Fluidkanal und der zweite Fluidkanal können ausgebildet sein, um die biologische Probe in die Kammer einzulassen und/oder aus der Kammer auszulassen. So kann die Befüllung der Kammer mit der aufzubereitenden Probe auf einfache und kostengünstige Weise bewerkstelligt werden. Auch können durch die Anlage eines Fluidzu- oder -abgangs für die beide Kammerabschnitte diese flexibler eingesetzt werden.

Gemäß einer besonderen Ausführungsform kann das zweite Substrat einen die zweite Kavität aufweisenden ersten Substratabschnitt und einen benachbart zu einem eine Wand der Kammer bildenden Boden der zweiten Kavität angeordneten zweiten Substratabschnitt aufweisen. Insbesondere kann dabei der erste Substratabschnitt aus Silizium gebildet sein und der zweite Substratabschnitt aus Glas gebildet sein. Diese Ausführungsform bietet zum einen den Vorteil, der Kostenersparnis, indem eine Wandung der zweiten Kavität als einfacher Durchbruch in dem Siliziumabschnitt gebildet werden kann und der Glasabschnitt einen Boden der zweiten Kavität bildet. Zum anderen gibt der außen angeordnete Substratabschnitt aus Glas einen Blick ins Innere der Kammer frei, beispielsweise um Vorgänge dort zu überwachen.

Gemäß einer Ausführungsform kann die elektrisch leitfähige Struktur eine erste Leiterbahn zwischen einem ersten Anschlussbereich der elektrisch leitfähigen Struktur und einem zweiten Anschlussbereich der elektrisch leitfähigen Struktur zum Anlegen einer ersten Spannung zwischen dem ersten Anschlussbereich und dem zweiten Anschlussbereich und eine zweite Leiterbahn zwischen einem dritten Anschlussbereich der elektrisch leitfähigen Struktur und einem vierten Anschlussbereich der elektrisch leitfähigen Struktur zum Anlegen einer zweiten Spannung zwischen dem dritten Anschlussbereich und dem vierten Anschlussbereich aufweisen. Insbesondere kann dabei die erste Leiterbahn in einem ersten Mäander entlang der Mehrzahl von Durchbrüchen des Filterelements von dem ersten Anschlussbereich zu dem zweiten Anschlussbereich verlaufen und die zweite Leiterbahn in einem zu dem ersten Mäander parallelen zweiten Mäander entlang der Mehrzahl von Durchbrüchen des Filterelements von dem dritten zu dem vierten Anschlussbereich verlaufen. Diese Ausführungsform erlaubt neben einem Anlegen unterschiedlicher Spannungen an die elektrisch leitfähige Struktur auch eine effektive Erwärmung des benachbart zu der elektrisch leitfähigen Struktur angeordneten Filterelements.

In einer Weiterbildung kann die erste Leiterbahn zumindest einen ersten Leiterbahnabschnitt aufweisen, und die zweite Leiterbahn zumindest einen zweiten Leiterbahnabschnitt aufweisen. Dabei kann sich der erste Leiterbahnabschnitt zu einem Rand des Durchbruches erstrecken und der zweite Leiterbahnabschnitt zu einem dem Rand gegenüberliegenden weiteren Rand des Durchbruches erstrecken. So kann ohne Weiteres ein über die jeden der Durchbrüche wirkendes elektrisches Feld auf dem Filterelement aufgebaut werden.

Ferner kann die Vorrichtung eine Kompensationsschicht für eine Spannungskompensation zwischen der ersten Leiterbahn und der zweiten Leiterbahn aufweisen. Die Kompensationsschicht kann dabei lateral angrenzend an die elektrisch leitfähige Struktur zwischen dem ersten Substrat und dem zweiten Substrat angeordnet sein. Mit dieser Ausführungsform kann ein unerwünschter Kurzschluss zwischen der ersten Leiterbahn und der zweiten Leiterbahn bzw. zwischen den Leiterbahnen und die Vorrichtung eventuell umgebenden stromführenden Strukturen auf einfache Weise vermieden werden.

Für eine elektrische Kontaktierung der elektrisch leitfähigen Struktur kann das erste Substrat einen ersten Durchtritt zwischen der Hauptseite des ersten Substrats und der elektrisch leitfähigen Struktur aufweisen und zusätzlich oder alternativ das zweite Substrat einen zweiten Durchtritt zwischen einer von der zweiten Kavität abgewandten Hauptseite des zweiten Substrats und der elektrisch leitfähigen Struktur aufweisen. Unter einem Durchtritt kann ein weiterer Durchbruch oder eine Öffnung verstanden werden. Mit dieser Ausführungsform kann eine elektrische Anbindung der elektrisch leitfähigen Struktur an eine außerhalb der Vorrichtung gelegene Spannungsquelle auf einfache Weise bewerkstelligt werden. Wahlweise kann einer der Durchtritte oder können beide Durchtritte metallisiert sein, um noch vielfältigere Anschlussmöglichkeiten zu schaffen.

Gemäß einer Ausführungsform kann die Vorrichtung eine weitere leitfähige Struktur aufweisen, die an dem Boden der zweiten Kavität angeordnet sein kann. Diese Ausführungsform erweitert die Anwendungsmöglichkeiten der Vorrichtung, so kann eine der leitfähigen Strukturen zu Heizzwecken, beispielsweise zur Durchführung einer PCR, und die andere z. B. für die elektrische Lyse eingesetzt werden.

Ferner kann die Vorrichtung einen Lysataufnahmebereich zum Aufnehmen eines bei der Lyse der organischen Zellen gewonnenen Lysats aufweisen. Der Lysataufnahmebereich kann benachbart zu dem ersten Fluidkanal und/oder dem zweiten Fluidkanal an der Hauptseite des ersten Substrats angeordnet sein. Mit dieser Ausführungsform können in der Vorrichtung lysierte Zellbestandteile vorteilhafterweise auf kürzestem Wege einer weiterführenden Diagnose zugeführt werden. Die mit langen Transportwegen einhergehenden Probleme wie eine Verringerung der Lysatmenge aufgrund z. B. Totvolumens oder eine Verschmutzung des Lysats können so auf einfache Weise vermieden werden.

Es wird weiterhin ein Analysesystem zum Analysieren einer biologischen Probe vorgestellt, wobei das Analysesystem die folgenden Merkmale aufweist:
eine Vorrichtung zum Aufbereiten der biologischen Probe organischer Zellen gemäß einer der hier vorgestellten Variante; und
eine Detektionseinheit zum Detektieren eines in der Vorrichtung aus den organischen Zellen herausgelösten vorbestimmten Zellbestandteils der biologischen Probe, wobei die Detektionseinheit fluidisch mit der Vorrichtung gekoppelt ist.

Gemäß einer Ausführungsform kann die Detektionseinheit einen Schichtaufbau aus einem ein Kanalsystem aufweisenden ersten Polymersubstrat, einem eine Ausnehmung aufweisenden zweiten Polymersubstrat und einer zwischen dem ersten Polymersubstrat und dem zweiten Polymersubstrat angeordneten, ein mikrofluidisches Netzwerk aufweisenden, Mikrofluidschicht sowie eine mit dem Kanalsystem gekoppelte Analysekavität zur Durchführung eines Analyseverfahrens an dem in der Vorrichtung gewonnen Lysat aufweisen. Dabei kann die Vorrichtung derart in der Ausnehmung und angrenzend an die Mikrofluidschicht angeordnet sein, dass der Lysataufnahmebereich in der Analysekavität liegt und der erste Fluidkanal und/oder der zweite Fluidkanal fluidisch mit dem Kanalsystem gekoppelt ist. Diese Ausführungsform ermöglicht eine Realisierung des Analysesystems mit geringen Kosten, da die einfach aufgebaute und flexibel einsetzbare Detektionseinheit mit geringen Herstellungskosten in großer Stückzahl gefertigt werden kann.

Gemäß einer Ausführungsform kann die Analysekavität durch eine Durchgangsöffnung in der Mikrofluidschicht gebildet sein. So kann das Analysesystem besonders bauraumsparend realisiert werden.

Gemäß einer weiteren Ausführungsform kann die Analysekavität durch eine Aushöhlung in dem ersten Polymersubstrat gebildet sein. In dieser Ausführung kann die Analysekavität besonders einfach mit dem Kanalsystem des ersten Polymersubstrats gekoppelt werden.

Ferner wird ein Verfahren zum Aufbereiten einer biologischen Probe vorgestellt, wobei das Verfahren den folgenden Schritt aufweist:
Bewegen der biologischen Probe zwischen einer in einem ersten Substrat angelegten, einen ersten Kammerabschnitt einer Kammer zum Aufnehmen der biologischen Probe bildenden, ersten Kavität und einer in einem zweiten Substrat angelegten, einen zweiten Kammerabschnitt der Kammer bildenden, zweiten Kavität durch ein zwischen der ersten Kavität und der zweiten Kavität ausgebildetes Filterelement mit einer Mehrzahl von Durchbrüchen und eine an dem Filterelement angeordnete elektrisch leitfähige Struktur, um eine Mehrzahl von organischen Zellen der biologischen Probe auf der Mehrzahl von Durchbrüchen zurückzuhalten; und
Anlegen zumindest einer elektrischen Spannung an die elektrisch leitfähige Struktur in Anwesenzeit eines in die Kammer eingespülten Lysemittels, um die an der Mehrzahl von Durchbrüchen zurückgehaltene Mehrzahl von organischen Zellen zu lysieren.

Das Verfahren kann in einer Vorrichtung zum Aufbereiten einer biologischen Probe gemäß einer der oben erläuterten Ausführungsformen ausgeführt werden. Auch durch diese Ausführungsvariante der Erfindung in Form eines Verfahrens kann die der Erfindung zugrunde liegende Aufgabe schnell und effizient gelöst werden.

Gemäß einer Ausführungsform des Verfahrens kann in dem Schritt des Anlegens die elektrische Spannung über eine einen ersten Anschlussbereich der elektrisch leitfähigen Struktur mit einem zweiten Anschlussbereich der elektrisch leitfähigen Struktur verbindende erste Leiterbahn angelegt werden, um die an der Mehrzahl von Durchbrüchen zurückgehaltene Mehrzahl von organischen Zellen basierend auf einer Joulschen Erwärmung des Filterelements thermisch zu lysieren. Auf diese Weise kann an mehreren Durchbrüchen eine technisch einfach umzusetzende Erwärmung vorgenommen werden.

Gemäß einer weiteren Ausführungsform kann in dem Schritt des Anlegens eine weitere elektrische Spannung über eine einen dritten Anschlussbereich der elektrisch leitfähigen Struktur mit einem vierten Anschlussbereich der elektrisch leitfähigen Struktur verbindende zweite Leiterbahn angelegt werden, um die an der Mehrzahl von Durchbrüchen zurückgehaltene Mehrzahl von organischen Zellen basierend auf einer Erzeugung eines elektrischen Feldes zwischen der ersten Leiterbahn und der zweiten Leiterbahn elektrisch zu lysieren. Eine solche Ausführungsform des hier vorgestellten Ansatzes bietet den Vorteil einer besonders effizienten Lyse von Zellen in den Durchbrüchen bei einem geringen Bauraumbedarf der verwendeten Lysestruktur.

Weiterhin kann das Verfahren einen Schritt des erneuten Anlegens der elektrischen Spannung an die elektrisch leitfähige Struktur aufweisen. So kann in Anwesenheit eines Reaktionsmittels in der Kammer eine Mehrzahl von bei dem Lysieren aus der Mehrzahl von organischen Zellen freigesetzten Zellbestandteilen vervielfältigt werden. Da mit dieser Ausführungsform ein Ortswechsel zwischen Lyse und Vervielfältigung der zu untersuchenden Substanzen vermieden werden kann, können die Gefahren einer Verunreinigung und oder eines Verlusts eines Teils des Lysats aufgrund von Totvolumen bei der Weiterleitung ausgeschaltet werden.

Der hier vorgestellte Ansatz wird nachstehend anhand der beigefügten Zeichnungen beispielhaft näher erläutert. Es zeigen:
Fig. 1 bis 5 Querschnittansichten einer Vorrichtung zum Aufbereiten einer biologischen Probe, gemäß Ausführungsbeispielen der vorliegenden Erfindung;
Fig. 6 eine Aufsicht auf eine elektrisch leitfähige Struktur einer Vorrichtung zum Aufbereiten einer biologischen Probe, gemäß einem Ausführungsbeispiel der vorliegenden Erfindung;
Fig. 7 und 8 Querschnittansichten eines Analysesystems zum Analysieren einer biologischen Probe, gemäß Ausführungsbeispielen der vorliegenden Erfindung;
Fig. 9 ein Ablaufdiagramm eines groben Prozessablaufs eines Verfahrens zum Aufbereiten einer biologischen Probe, gemäß einem Ausführungsbeispiel der vorliegenden Erfindung;
Fig. 10 ein Ablaufdiagramm eines Prozessabschnitts der Filtration des Verfahrens aus Fig. 9, gemäß einem Ausführungsbeispiel der vorliegenden Erfindung;
Fig. 11 ein Ablaufdiagramm eines Prozessabschnitts der elektrischen und/oder thermischen Lyse des Verfahrens aus Fig. 9, gemäß einem Ausführungsbeispiel der vorliegenden Erfindung; und
Fig. 12 ein Ablaufdiagramm eines Prozessabschnitts der Amplifikation von Zielzellen des Verfahrens aus Fig. 9, gemäß einem Ausführungsbeispiel der vorliegenden Erfindung.

In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

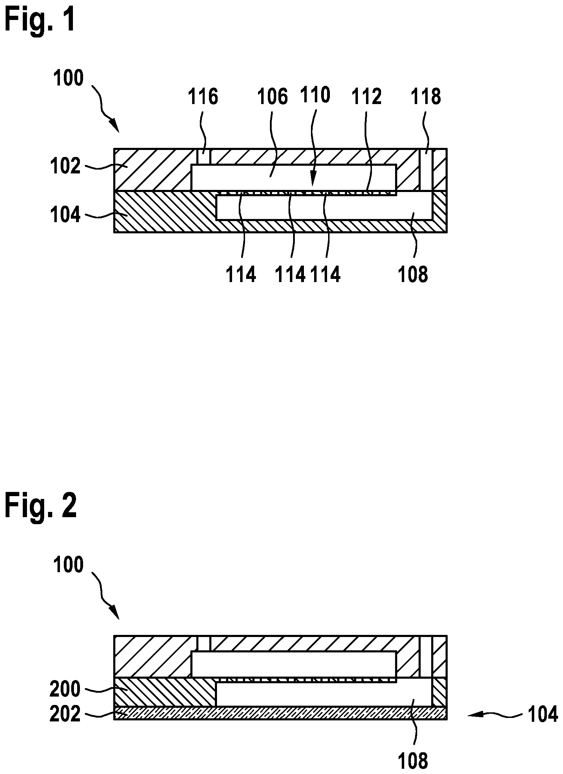

Fig. 1 zeigt einen Querschnitt durch ein beispielhaftes grundlegendes Ausführungsbeispiel einer hierin vorgestellten Vorrichtung 100 zum Aufbereiten einer biologischen Probe (die auch als Analysenprobe bezeichnet werden kann). Es handelt sich bei der Vorrichtung 100 um einen Schichtaufbau aus zwei Substraten 102 und 104 mit je einer Ausnehmung bzw. Kavität 106 bzw. 108 sowie einer hier nicht gezeigten elektrisch leitfähigen Struktur. Die Kavitäten 106 bzw. 108 bilden Teilkammern bzw. Kammerabschnitte einer gemeinsamen Kammer 110 zum Aufnehmen der biologischen Probe in der Vorrichtung 100. Ein Filterelement 112 in Form einer Membran mit einer Mehrzahl von Durchbrüchen bzw. Filterporen 114 ist bei dem in Fig. 1 gezeigten Ausführungsbeispiel der Vorrichtung 100 innerhalb des zweiten Substrats 104 ausgebildet, kann jedoch auch innerhalb des ersten Substrats 102 ausgebildet sein. Die Ausnehmungen bzw. Teilkammern 106 und 108 weisen je einen fluidischen Zugang in Form eines ersten Fluidkanals 116 und eines zweiten Fluidkanals 118 auf.

Die Substrate 102, 104 bestehen bei dem gezeigten Ausführungsbeispiel der Vorrichtung 100 aus Silizium. Zur Herstellung der Ausnehmungen 106, 108 und Filterporen 114 wurde ein waferbasierter Ätzprozess wie Trockenätzen, z. B. reaktives lonentiefenätzen bzw. DRIE (englisch: Deep Reactive Ion Etching) oder alternativ ein nasschemisches Verfahren, z. B. KOH-Ätzen, eingesetzt. Zum Verbinden der Substratschichten 102, 104 kam ein übliches Waferbondverfahren, z. B. anodisches Bonden, eutektisches Bonden oder Silizium-Fusion Bonden, zum Einsatz.

Die Vorrichtung 100 wird beispielsweise eingesetzt, um eine Probe organischen Materials wie beispielsweise eine Blutprobe für eine nachfolgende Analyse aufzubereiten, z. B. im Hinblick auf in der Probe enthaltene Pathogene. So kann eine Größe der in dem Filterelement 112 ausgebildeten Durchbrüche 114 geeignet bemessen sein, um in der Probe enthaltenen Zielzellen an dem Filterelement 112 zurückzuhalten und damit aus der Probe herauszufiltern. Die so an der Filtermembran 112 konzentrierten Zielzellen können anschließend unter Einsatz der in Fig. 1 nicht gezeigten elektrisch leitfähigen Struktur lysiert werden, beispielsweise, um eine für die Analyse benötigte Nukleinsäure aus dem Zellmaterial herauszulösen. Die Lyse der Zielzellen kann mittels Anlegen einer elektrischen Spannung an die elektrisch leitfähige Struktur in Form einer thermischen und/oder einer elektrischen Lyse vollzogen werden. Da in der Vorrichtung 100 alle Schritte der Zellaufbereitung in derselben Kammer durchgeführt werden, kann die Vorrichtung 100 auch als sogenanntes Einkammer-LoC bezeichnet werden.

Fig. 2 zeigt einen Querschnitt durch ein weiteres Ausführungsbeispiel der Vorrichtung 100, das durch die Besonderheit gekennzeichnet ist, dass das zweite Substrat 104 sich aus zwei Schichten bzw. Substratabschnitten 200 und 202 zusammensetzt. Diese beispielhafte Ausführung der Vorrichtung 100 hat insbesondere den Vorteil, dass für die Strukturierung der zweiten Kavität 108 in dem ersten Substratabschnitt 200 kostengünstigere Prozessschritte verwendet werden können, während der zweite Substratabschnitt 202 aus einem unstrukturierten Wafer besteht. Beispielsweise kann der zweite Substratabschnitt 202 auch aus Glas bestehen. Der hierdurch entstehende optische Zugang kann beispielsweise zur Prozesskontrolle eingesetzt werden.

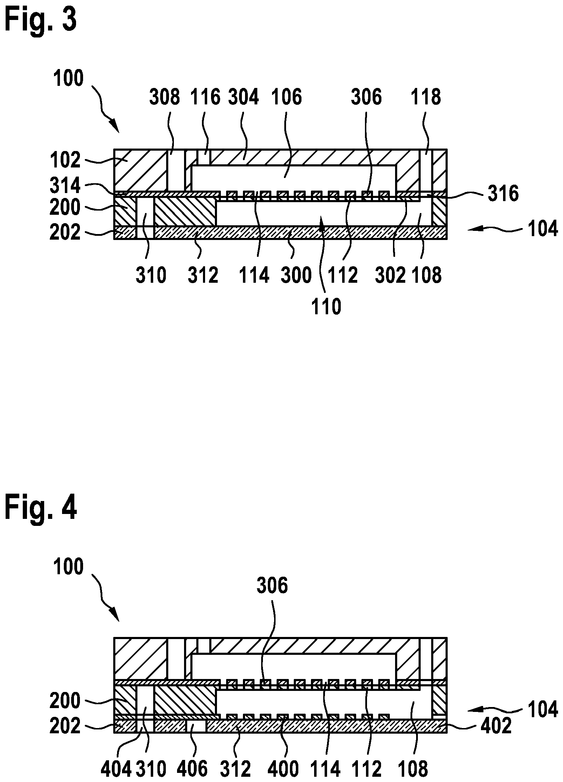

Fig. 3 zeigt die Vorrichtung 100 anhand eines weiteren beispielhaften Querschnitts ausführlicher. Wie die Darstellung zeigt, können (ohne Beschränkung der Allgemeinheit) das erste Substrat 102 und das zweite Substrat 104 als gleich dicke Schichten bzw. Platten ausgebildet sein, wobei sich auch bei dem hier gezeigten Ausführungsbeispiel das zweite Substrat 104 aus dem ersten Substratabschnitt 200, der die zweite Kavität 108 aufweist, und dem zweiten Substratabschnitt 202, der hier einen Boden der Vorrichtung 100 bildet, zusammensetzt. Eine der Kammer 110 zugewandte Seite des zweiten Substratabschnitts 202 bildet somit eine - hier die Kammer 110 nach unten begrenzende - Wand 300 der Kammer 110. Die durch die Kavitäten 106, 108 gebildeten Kammerabschnitte der Kammer 110 sind versetzt zueinander angeordnet, sodass ein Teilbereich der ersten Kavität 106 einem massiven Bereich des zweiten Substrats 104 gegenüberliegt und ein Teilbereich der zweiten Kavität 108 einem massiven Bereich des ersten Substrats 102 gegenüberliegt. Das Filterelement 112 wird durch einen im Herstellungsprozess der zweiten Kavität 108 stehen gelassenen Abschnitt einer dem ersten Substrat 102 zugewandten Hauptseite 302 des zweiten Substrats 104 gebildet. Unter einer Hauptseite ist hierin eine Seite der den Schichtaufbau 100 bildenden Elemente 102, 104 mit den größten Abmessungen zu verstehen. Aufgrund der sehr geringen Dicke dieses stehen gelassenen Abschnitts und der Vielzahl der im Herstellungsprozess erzeugten Durchbrüche ist das Filterelement 112 hier als eine Membran ausgebildet.

Wie die Darstellung in Fig. 3 zeigt, verläuft der erste Fluidkanal 116 zwischen einer von der ersten Kavität 106 abgewandten Hauptseite 304 des ersten Substrats 102 und der ersten Kavität 106. Der zweite Fluidkanal 118 erstreckt sich von der Hauptseite 304 des ersten Substrats 102 durch den der zweiten Kavität 108 gegenüberliegenden massiven Bereich des ersten Substrats 102 zu der zweiten Kavität 108. Im gezeigten Ausführungsbeispiel wird die biologische Probe über den ersten Fluidkanal 116 in den ersten Kammerabschnitt 106 der Kammer 110 eingelassen, strömt durch das Filterelement 112 in den zweiten Kammerabschnitt 108 der Kammer 110 und wird über den zweiten Fluidkanal 118 wieder aus der Kammer 110 herausgeführt, beispielsweise unter Einsatz einer Pumpe.

Insbesondere zeigt Fig. 3 eine zwischen dem ersten Substrat 102 und dem zweiten Substrat 104 angeordnete elektrisch leitfähige Struktur 306, die bei dem in Fig. 3 gezeigten Ausführungsbeispiel aus Metall besteht. Es können gemäß Ausführungsbeispielen auch andere elektrisch leitfähige Materialien verwendet werden. Wie die Darstellung in Fig. 3 zeigt, grenzt die elektrisch leitfähige Struktur 306 an das Filterelement 112 - hier an eine Oberseite der aus dem zweiten Substrat 104 herausgebildeten Filtermembran 112 - an und erstreckt sich parallel zu den Hauptseiten 302 und 304 so, dass die Durchbrüche 114 für die Filterfunktion der Membran 112 freigestellt bleiben. Das erste Substrat 102 weist einen ersten Durchtritt 308 zwischen der Hauptseite 304 des ersten Substrats 102 und der elektrisch leitfähigen Struktur 306 auf. Das zweite Substrat 104 weist einen zweiten Durchtritt 310 zwischen einer von der zweiten Kavität 108 abgewandten Hauptseite 312 des zweiten Substrats 104 und der elektrisch leitfähigen Struktur 306 auf. Über die Durchtritte 308, 310 erfolgt eine elektrische Kontaktierung der elektrisch leitfähigen Struktur 306, um diese mit elektrischer Spannung zu versorgen.

Ferner weist die in Fig. 3 gezeigte beispielhafte Vorrichtung 100 eine Kompensationsschicht 314 auf. Die Kompensationsschicht 314 ist lateral an die elektrisch leitfähige Struktur 306 angrenzend zwischen dem ersten Substrat 102 und dem zweiten Substrat 104 angeordnet und weist für einen Durchtritt des Probenfluids im Bereich des zweiten Fluidkanals 118 eine Öffnung 316 auf.

Eine Dicke der elektrisch leitfähigen Schicht 306 beträgt gemäß Ausführungsbeispielen der Vorrichtung 100 10 nm bis 10 µm, bevorzugt 100 nm bis 1 µm. Im Falle einer sehr dünnen elektrisch leitfähigen Schicht 306 können übliche Waferbondverfahren weiterhin ohne die zusätzliche Kompensationsschicht 314 eingesetzt werden. Für eine dickere elektrisch leitfähige Schicht 306 wird, wie in Fig. 3 beispielhafte gezeigt, die zusätzliche Kompensationsschicht 314 eingeführt. Die Kompensationsschicht 314 ist beispielsweise aus Glass-Seal gebildet.

Für die Kontaktierung der elektrisch leitfähigen Schicht 306 ist je nach Ausführung der elektrisch leitfähigen Schicht 306 einer der beiden Zugänge 308, 310 ausreichend, sodass auf den anderen verzichtet werden kann. Für die Kontaktierung der elektrisch leitfähigen Schicht 306 können beispielsweise Federkontaktstifte eingesetzt werden. Der Zugang 308 hat den Vorteil, dass ein zum Anlegen des Zugangs 308 erforderlicher Durchbruch im ersten Substrat 102 leicht hergestellt werden kann, da dieser sich durch das gesamte Bauteil 102 erstreckt. Der Zugang 310 hat den Vorteil, dass bei einer eventuell nachfolgenden Integration der Vorrichtung 100 in eine polymere mikrofluidische Umgebung ein in den zweiten Durchtritt 310 eingesetzter Federkontaktstift in Richtung einer Bond-Verbindung zum mikrofluidischen Bauteil wirkt und dadurch die mechanische Belastung auf die Bond-Verbindung sinkt.

Gemäß alternativen Ausführungsbeispielen der Vorrichtung 100 können Wände der Durchtritte 308, 310 über elektrischen Kontaktstellen der elektrisch leitfähigen Schicht 306 mit einem elektrisch leitfähigen Material verfüllt sein, sodass eine neue Kontaktstelle auf den Außenseiten 304 bzw. 312 des Bauteils 100 entsteht. Dies hat insbesondere den Vorteil, dass außer Federkontaktstiften noch weitere elektrische Schnittstellen zur Kontaktierung der elektrisch leitfähigen Schicht 306 eingesetzt werden können, beispielsweise Schleifkontakte.

Fig. 4 zeigt einen Querschnitt eines weiteren Ausführungsbeispiels der Vorrichtung 100. Dieses Ausführungsbeispiel weist die Besonderheit einer weiteren elektrisch leitfähigen Struktur 400 und einer weiteren Kompensationsschicht 402 auf. Bei dem in der Darstellung in Fig. 4 gezeigten Ausführungsbeispiel der Vorrichtung 100 ist die weitere elektrisch leitfähige Struktur 400 zwischen dem ersten Substratabschnitt 200 und dem zweiten Substratabschnitt 202 des zweiten Substrats 104 angeordnet und grenzt an die den Boden 300 der zweiten Kavität 108 bildende Hauptseite des zweiten Substratabschnitts 202 an. Die weitere Kompensationsschicht 402 ist lateral an die weitere elektrisch leitfähige Struktur 400 angrenzend zwischen dem ersten Substratabschnitt 200 und dem zweiten Substratabschnitt 202 des zweiten Substrats 104 angeordnet und weist für die elektrische Kontaktierung der ersten elektrisch leitfähigen Struktur 306 im Bereich des zweiten Durchtritts 310 eine weitere Öffnung 404 auf.

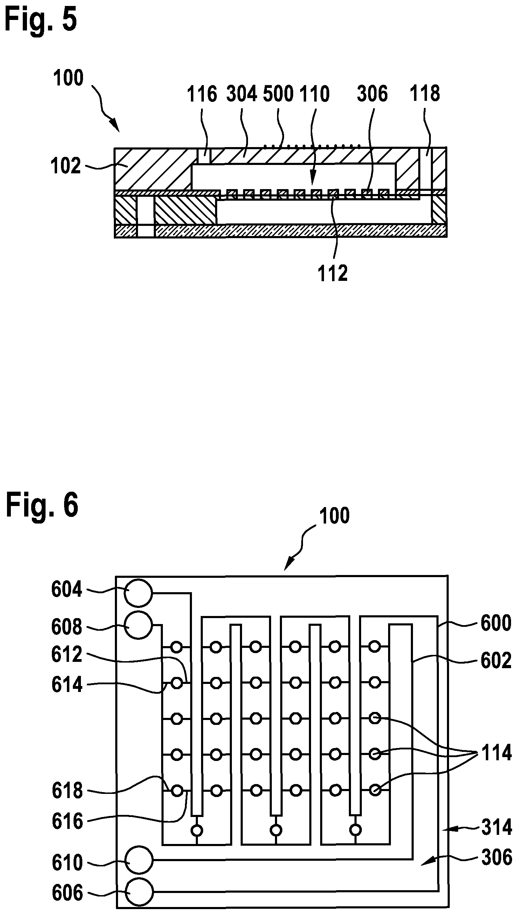

Im in Fig. 4 gezeigten Ausführungsbeispiel der Vorrichtung 100 wird auf den ersten elektrischen Zugang zur Kontaktierung der elektrisch leitfähigen Struktur 306 verzichtet. Stattdessen liegt zur Kontaktierung der weiteren elektrisch leitfähigen Struktur 400 ein dritter Durchtritt 406 zwischen der von der zweiten Kavität 108 abgewandten Hauptseite 312 des zweiten Substrats 104 und der weiteren elektrisch leitfähigen Struktur 400 vor. Die in Fig. 4 gezeigte beispielhafte Ausführung der Vorrichtung 100 ist durch den Vorteil gekennzeichnet, dass beispielsweise eine Heizfunktion zum Beheizen des Filterelements 112 auf die weitere bzw. untere Struktur 400 ausgelagert werden kann und sich damit eine höhere Designfreiheit für die elektrisch leitfähige Schicht 306 ergibt. Damit ist es beispielsweise möglich, eine sehr hohe Porendichte der Filtermembran 112 einzusetzen und trotzdem an jeder Pore bzw. jedem Durchbruch 114 einen Leiterbahnbereich für eine Lyse mit elektrischen Feldern zu positionieren. Auf die Leiterbahnbereiche wird anhand der nachfolgenden Fig. 6 noch genauer eingegangen.

Fig. 5 zeigt anhand einer weiteren Querschnittdarstellung eine besondere Ausführung, in der die Vorrichtung 100 um einen Detektionsbereich 500 zum Aufnehmen eines bei einer Lyse von Zellen in der Kammer 110 gewonnenen Lysats erweitert wurde. Wie die Darstellung in Fig. 5 zeigt, befindet sich der Detektionsbereich 500 zwischen dem ersten Fluidkanal 116 und dem zweiten Fluidkanal 118 an der Außenseite bzw. Hauptseite 304 des ersten Substrats 102. Der Detektionsbereich bzw. Detektionsbereich 500 wird im gezeigten Ausführungsbeispiel durch biochemische Fängermoleküle gebildet, die auf der Hauptseite 304 immobilisiert wurden. Bei den biochemischen Fängermolekülen handelt es sich z. B. um Proteine, Antikörper oder DNA in Form eines Microarrays.

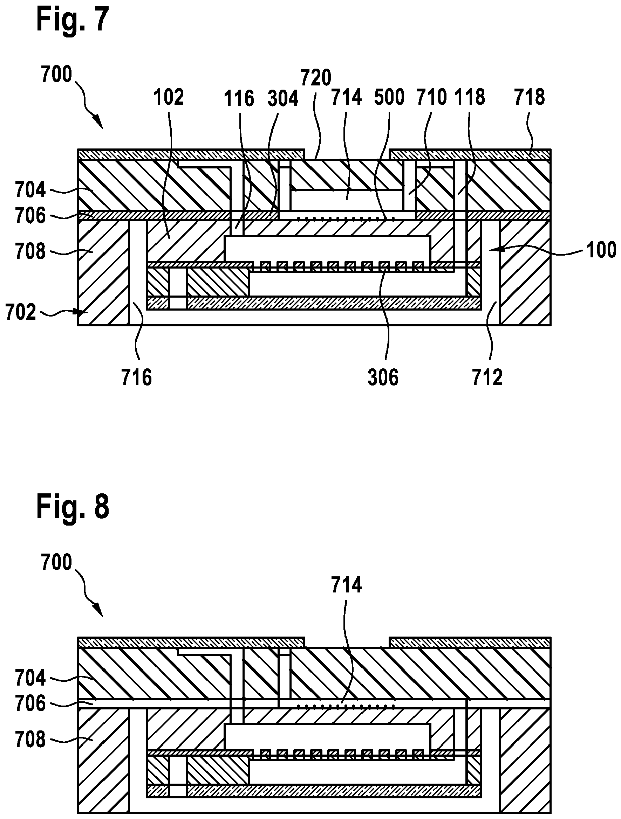

Diese in Fig. 5 gezeigte beispielhafte Ausführung der Vorrichtung 100 hat insbesondere den Vorteil, dass in Kombination mit einer entsprechenden mikrofluidischen Umgebung, wie sie nachfolgend anhand der Figuren 7 und 8 vorgestellt wird, eine in der Kammer 110 prozessierte Flüssigkeit aus dem Filter 112 über einen der beiden Fluidkanäle 116, 118 direkt auf den Detektionsbereich 500 gegeben werden kann. Hierdurch ergibt sich zum einen ein sehr kurzer Transportweg der Flüssigkeit. Zum anderen kann die im Bauteil 100 integrierte Heizfunktion - hier über die elektrisch leitfähige Schicht 306 - genutzt werden, um eine geeignete Temperatur für eine Detektionsreaktion der Flüssigkeit einzustellen.

Eine beispielhafte Dicke der Substrate 102 und 104 der in den Figuren 1 bis 5 gezeigten Ausführungsbeispiele der Vorrichtung 100 beträgt 10 bis 3000 µm, bevorzugt 100 bis 1000 µm. Eine beispielhafte Dicke der Filtermembran 112 liegt zwischen 0,1 und 500 µm, bevorzugt zwischen 10 und 200 µm. Eine beispielhafte laterale Abmessung der Filtermembran 112 beträgt 1 bis 20 mm, bevorzugt 5 bis 10 mm. Ein beispielhafter Durchmesser der Durchbrüche bzw. Poren 114 des Filterelements 112 liegt zwischen 0,1 und 100 µm, bevorzugt zwischen 0,2 und 20 µm. Eine beispielhafte Dichte der Poren 114 liegt zwischen 10^5 und 10^9 Poren pro Quadratzentimeter der Filtermembran 112.

Fig. 6 zeigt einen Längsschnitt durch ein Ausführungsbeispiel der Vorrichtung 100, der eine Aufsicht auf die aus der elektrisch leitfähigen Struktur 306 und der Kompensationsschicht 314 gebildete Ebene des Schichtaufbaus erlaubt. Wie die Darstellung in Fig. 6 zeigt, umfasst die elektrisch leitfähige Struktur 306 eine erste Leiterbahn 600 und eine zweite Leiterbahn 602. Die erste Leiterbahn 600 verläuft in einem ersten Mäander zwischen einem ersten Anschlussbereich 604 und einem zweiten Anschlussbereich 606 der elektrisch leitfähigen Struktur 306. Die zweite Leiterbahn 602 verläuft in einem zu dem ersten Mäander parallelen zweiten Mäander zwischen einem dritten Anschlussbereich 608 und einem vierten Anschlussbereich 610 der elektrisch leitfähigen Struktur 306. An die erste Leiterbahn 600 wird zwischen dem ersten Anschlussbereich 604 und dem zweiten Anschlussbereich 606 eine erste Spannung angelegt, und an die zweite Leiterbahn 602 wird zwischen dem dritten Anschlussbereich 608 und dem vierten Anschlussbereich 610 eine zweite Spannung angelegt. Die erste und zweite Spannung können gleich oder unterschiedlich sein.

Die elektrisch leitfähige Struktur 306 ist so in die Kompensationsschicht 314 eingebettet bzw. von dieser lateral umgeben, dass die hier unterhalb der Schichten 306 und 314 angeordnete Filtermembran vollständig abgedeckt ist, wobei geeignet positionierte Öffnungen der Kompensationsschicht 314 die Durchbrüche 114 des Filterelements zum Durchtritt der Probenflüssigkeit frei geben.

Insbesondere ist aus der Darstellung in Fig. 6 ersichtlich, dass die erste Leiterbahn 600 und die zweite Leiterbahn 602 entlang Reihen der rasterförmig angelegten Durchbrüche 114 des hier unterhalb der elektrisch leitfähigen Schicht 306 angeordneten Filterelements der Vorrichtung 100 verlaufen. Die erste Leiterbahn 600 verläuft auf einer Seite jeder Reihe von Durchbrüchen 114 in einem vorbestimmten Abstand zu einem Randbereich 612 je eines der Durchbrüche 114, und die zweite Leiterbahn 602 verläuft auf der anderen Seite jeder Reihe von Durchbrüchen 114 in demselben vorbestimmten Abstand zu einem dem Randbereich 612 gegenüberliegenden weiteren Randbereich 614 je eines der Durchbrüche 114.

Bei dem in Fig. 6 gezeigten Ausführungsbeispiel der Vorrichtung 100 weist die erste Leiterbahn 600 eine Mehrzahl von ersten Leiterbahnabschnitten 616 auf und die zweite Leiterbahn 602 eine Mehrzahl von zweiten Leiterbahnabschnitten 618 auf, wobei Mehrzahl der ersten Leiterbahnabschnitte 616 und die Mehrzahl der zweiten Leiterbahnabschnitte 618 in ihrer Zahl einer Zahl der Mehrzahl von Durchbrüchen 114 des Filterelements entsprechen. Wie die Darstellung in Fig. 6 zeigt, erstreckt sich je einer der ersten Leiterbahnabschnitte 616 zu dem Randbereich 612 je eines der Durchbrüche 114 und je einer der zweiten Leiterbahnabschnitte 618 zu dem weiteren Randbereich 614 je eines der Durchbrüche 114.

Die über der Filtermembran angeordnete elektrisch leitfähige Schicht 306 besteht bei dem in Fig. 6 gezeigten Ausführungsbeispiel der Vorrichtung 100 aus einem Metall, insbesondere aus Kupfer, Aluminium, Titan, Platin oder Gold. Die elektrisch leitfähige Schicht 306 ist derart strukturiert, dass sich die vier Anschlussbereiche 604, 606, 608 und 610 ergeben. Die erste Leiterbahn 600 ist dabei so strukturiert, dass die Anschlussbereiche 604 und 606 elektrisch miteinander verbunden sind. Die weitere bzw. zweite Leiterbahn 602 verbindet die Anschlussbereiche 608 und 610. Gleichzeitig sind die Leiterbahnen 600 und 602 so zwischen den Durchbrüchen bzw. Poren 114 der Filtermembran verlegt, dass sich eine möglichst lange Strecke mit einem hohen elektrischen Widerstand ergibt. Auf diese Weise kann durch Anlegen einer Spannung zwischen den Anschlussbereichen 604 und 606 bzw. 608 und 610 durch die Erzeugung von Joulscher Wärme eine Heizfunktion in der elektrisch leitfähigen Schicht 306 zum Beheizen des angrenzenden Filterelements realisiert werden, beispielsweise zur thermischen Lyse gefilterter Zellen oder zum Heizen des gesamten Systems 100 für eine PCR-Reaktion.

Durch Kurzschließen der Anschlussbereiche 604 und 606 und der Anschlussbereiche 608 und 610 und dem anschließenden Anlegen einer Spannung zwischen den kombinierten Bereichen 604, 606 und 608, 610 lässt sich ein elektrisches Feld zwischen jeweils gegenüberliegenden Leiterbahnbereichen bzw. Leiterbahnabschnitten 616 und 618 erzeugen, welches sich über jeweils eine Pore 114 erstreckt. Mithilfe dieses elektrischen Feldes ist es möglich, eine im Bereich der Pore 114 befindliche Zelle zu lysieren. Hierbei können wahlweise sowohl statische elektrische Felder als auch elektromagnetische Wechselfelder mit Feldstärken von 1 kV/cm bis 1000 kV/cm verwendet werden. Die in Fig. 6 gezeigte Art der Strukturierung der elektrisch leitfähigen Schicht 306 ist besonders vorteilhaft, da durch den geringen Abstand der Leiterbahnbereiche 616, 618 auch mit niedrigen Spannungen sehr hohe Felder erzeugt werden können. Es ist weiterhin ein Mischbetrieb der beiden beschriebenen Lyse-Verfahren möglich, d. h. eine thermische Lyse in Kombination mit einer Lyse mit elektrischen bzw. elektromagnetischen Feldern, wodurch die Lyseausbeute weiter gesteigert werden kann.

Durch geeignete Anpassung der Porengrößen der Durchbrüche 114 ist es bei dem in Fig. 6 im Längsschnitt gezeigten Aufbau 100 möglich, relevante Probenbestandteile, z. B. Partikel, insbesondere Zellen, beispielsweise Bakterien oder zirkulierende Tumorzellen bzw. CTCs (englisch: Circulating Tumor Cells) auf dem Filter zurückzuhalten. Die metallischen Strukturen 600, 602, 616, 618 können anschließend als Heizstrukturen zur Durchführung einer PCR genutzt werden.

Fig. 7 zeigt eine Querschnittansicht eines Ausführungsbeispiels eines hierin vorgeschlagenen Analysesystems 700 zum Analysieren einer biologischen Probe. Das Analysesystem 700 inkorporiert die Vorrichtung 100, insbesondere das anhand der Fig. 5 vorgestellte, um den Detektionsbereich 500 erweiterte, Ausführungsbeispiel der Vorrichtung 100. Neben der Vorrichtung 100 umfasst das Analysesystem 700 eine Detektionseinheit 702 zum Detektieren eines in der Vorrichtung 100 aus organischen Zellen herausgelösten vorbestimmten Zellbestandteils der biologischen Probe.

Wie die Darstellung in Fig. 7 zeigt, bildet die Detektionseinheit 702 einen Schichtaufbau aus einem ersten Polymersubstrat 704, einer Mikrofluidschicht 706 und einem zweiten Polymersubstrat 708. Das erste Polymersubstrat 704 umfasst ein Kanalsystem 710, das an die Fluidkanäle 116, 118 ankoppelt und somit die Vorrichtung 100 fluidisch mit der Detektionseinheit 702 verbindet. Die Mikrofluidschicht 706 ist zwischen dem ersten Polymersubstrat 704 und dem zweiten Polymersubstrat 708 angeordnet und weist ein in der Darstellung nicht explizit gezeigtes mikrofluidisches Netzwerk auf. Das zweite Polymersubstrat 708 weist eine zu der Mikrofluidschicht 706 gerichtete Ausnehmung 712 auf, in der die Vorrichtung 100 derart angeordnet ist, dass das erste Substrat 102 der Vorrichtung 100 an die Mikrofluidschicht 706 angrenzt. Ferner umfasst die Detektionseinheit 702 eine mit dem Kanalsystem 710 fluidisch gekoppelte Analysekavität 714 zur Durchführung eines Analyseverfahrens an einem in der Vorrichtung 100 gewonnen Lysat. Bei dem in Fig. 7 gezeigten Ausführungsbeispiel des Analysesystems 700 ist die Analysekavität 714 in Form einer Aushöhlung in dem ersten Polymersubstrat 704 ausgebildet. Wie aus der Darstellung in Fig. 7 gut ersichtlich, ist die Vorrichtung 100 derart in der Ausnehmung 712 und angrenzend an die Mikrofluidschicht 706 angeordnet, dass der Detektionsbereich 500 in der Analysekavität 714 liegt und der erste Fluidkanal 116 sowie der zweite Fluidkanal 118 fluidisch mit dem Kanalsystem 710 gekoppelt sind.

Das Polymersubstrat 704 ist z. B. aus PC, PP, PE, COP, COC oder PMMA gebildet und durch die Zwischenschicht bzw. Mikrofluidschicht 706 von dem weiteren Polymersubstrat 708 getrennt. Die Mikrofluidschicht 706 kann ebenfalls aus einem Polymer, insbesondere einem thermoplastischen Elastomer, bestehen bzw. als eine Schmelzklebefolie oder doppelseitige Klebefolie ausgebildet sein. In dem weiteren bzw. zweiten Polymersubstrat 708 befindet sich die Ausnehmung 712, die es erlaubt, das Bauteil bzw. die Vorrichtung 100 in Kontakt mit der Zwischenschicht 706 zu bringen. Dabei ist die Ausnehmung 712 so ausgelegt, dass um das Bauteil 100 herum ein Spalt 716 mit einer Breite von 0,1 mm bis 10 mm, bevorzugt von 2 mm bis 5 mm, entsteht. Der Spalt 716 erlaubt es, das Bauteil 100 mit einer von außen angebrachten Luftkühlung schnell abzukühlen. Hierdurch werden insbesondere in Kombination mit einer PCR schnelle Prozesszeiten erreicht.

Mithilfe geeigneter Verfahren, beispielsweise Laserdurchstrahlschweißen bzw.-bonden, Thermobonden oder Ultraschallschweißen, ist es möglich, die Vorrichtung 100 mechanisch fest und fluidisch dicht mit dem Schichtaufbau bestehend aus dem Polymersubstrat 704 und der Zwischenschicht 706 zu verbinden. Auf diese Weise kann das Bauteil 100 kostengünstig in den polymeren Schichtaufbau 702 integriert werden, welcher es erlaubt, mithilfe der flexiblen Zwischenschicht 706 mikrofluidische Funktionen wie Ventile und Pumpen bereitzustellen. Zur Verbesserung der Haftung zwischen der Vorrichtung 100 und der Zwischenschicht 706 können Oberflächenbehandlungen oder zusätzliche Hilfsschichten auf der Kontaktseite 304 der Vorrichtung 100 verwendet werden, beispielsweise Plasma- oder HDMS-Behandlungen sowie Fotolackschichten.

Innerhalb des ersten Polymersubstrats 704 befindet sich die Analysekavität 714, die derart an die mikrofluidischen Kanäle 710 angeschlossen ist, dass die Analysekavität 714 auf einer Seite mit dem mikrofluidischen Netzwerk der Zwischenschicht 706 verbunden ist und auf der anderen Seite mit dem Fluidkanal 118. Bei dem in Fig. 7 gezeigten Ausführungsbeispiel des Analysesystems 700 ist das Polymersubstrat 704 außen mit einer Schicht 718, z. B. einem Polymer oder einer Klebefolie, bedeckt, um die mikrofluidischen Kanäle 710 nach außen zu schließen. Besonders vorteilhaft ist ein optischer Zugang zur Kavität 714 vorgesehen, bei dem in Fig. 7 gezeigten Ausführungsbeispiel des Analysesystems 700 in Form einer Aussparung 720 in der Schicht 718. Gemäß einem alternativen Ausführungsbeispiel kann die Schicht 718 stattdessen im Bereich der Analysekavität 714 oder vollständig transparent ausgebildet sein.

Mit dem in Fig. 7 beispielhaft gezeigten Aufbau 700 ist es möglich, nach Fertigstellung aller Reaktionen innerhalb der Vorrichtung 100 beispielsweise mit einer (nicht gezeigten) mikrofluidischen Pumpe ein Reaktionsprodukt über den Fluidkanal 118 in Kontakt mit dem Detektionsbereich 500 zu bringen und eine Detektionsreaktion zu starten. In diesem Falle kann der im Bauteil 100 über die elektrisch leitfähige Schicht 306 integrierte Heizer genutzt werden, um eine geeignete Temperatur während der Detektionsreaktion bereitzustellen.

Fig. 8 zeigt in einer weiteren Querschnittansicht eine der in Fig. 7 gezeigten Ausführung ähnelnde weitere beispielhafte Ausführung des Analysesystems 700. Als hauptsächlicher Unterschied ist bei dem in Fig. 8 gezeigten Ausführungsbeispiel des Analysesystems 700 die Analysekavität 714 nicht in dem ersten Polymersubstrat 704 ausgenommen, sondern als eine Aussparung bzw. Durchgangsöffnung in der Mikrofluidschicht 706 gebildet. Diese Ausführung hat insbesondere den Vorteil, dass eine Höhe der Kavität 714 über eine Dicke der Zwischenschicht 706 sehr genau eingestellt werden kann. Insbesondere können sehr geringe Höhen realisiert werden, wodurch sich das Totvolumen reduziert und damit die Sensitivität der Detektionsreaktion gesteigert wird.

Eine beispielhafte Dicke der Polymersubstrate 704, 708 beträgt 0,1 bis 10 mm, bevorzugt 1 mm bis 3 mm, eine beispielhafte Dicke der Zwischenschicht 706 liegt zwischen 5 und 500 µm, bevorzugt zwischen 50 µm und 150 µm. Beispielhafte Kanalquerschnitte des Kanalsystems 710 betragen 10 x 10 µm² bis 3 x 3 mm², bevorzugt 100 x 100 pm² bis 1x 1 mm². Laterale Abmessungen des gesamten Analysesystems 700 lieben zwischen 10 x 10 und 200 x 200 mm², bevorzugt zwischen 30 x 30 mm² und 100 x 100 mm²

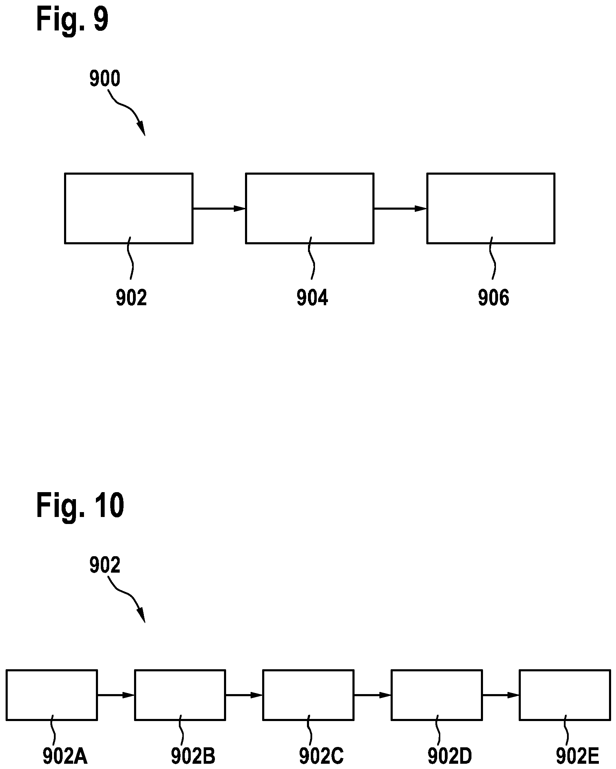

Fig. 9 zeigt einen schematischen Prozessablauf eines Ausführungsbeispiels eines Verfahrens 900 zum Aufbereiten einer biologischen Probe. Das Verfahren 900 kann in einem anhand der Figuren 1 bis 5 vorgestellten Einkammer-LoC mit einer in einer PCR-Kammer integrierten Filtermembran ausgeführt werden. Die Kammer besitzt einen fluidischen Ein - und Auslass und zusätzlich eine elektrisch leitfähige Schicht. Stark verkürzt ausgedrückt läuft das Verfahren 900 so ab, dass eine fluidische Probe durch den in der Kammer befindlichen Filter gespült wird, um Zielzellen herauszufiltern und zu fixieren. Nach Waschen des Filters wird ein PCR-Reaktionsmix eingespült und die fluidischen Ein- und Ausgänge der Kammer verschlossen. Die auf dem Filter befindlichen Zellen werden mittels der Elektroden entweder thermisch oder elektrisch lysiert. DNA-Abschnitte aus den aufgeschlossenen Zellen werden anschließend in der gleichen Kammer amplifiziert. Die dafür benötigten Temperaturzyklen werden von den als resistive Heizer fungierenden Elektroden bereitgestellt.

Das Verfahren 900 lässt sich grob in drei relevante Prozessabschnitte unterteilen. Ein erster Prozessabschnitt des Isolierens von Zellen bzw. Zielzellen aus der biologischen Probe umfasst einen Schritt 902 des Bewegens der biologischen Probe von einem ersten Kammerabschnitt der Kammer durch ein zwischen dem ersten Kammerabschnitt und einem zweiten Kammerabschnitt ausgebildetes Filterelement und eine an dem Filterelement angeordnete elektrisch leitfähige Struktur in den zweiten Kammerabschnitt der Kammer, um eine Mehrzahl von Zellen der biologischen Probe auf einer Mehrzahl von Durchbrüchen in dem Filterelement zurückzuhalten. Ein zweiter Prozessabschnitt des Aufschließens bzw. Lysierens der Zielzellen umfasst einen Schritt 904 des Anlegens einer elektrischen Spannung an die an dem Filterelement angeordnete elektrisch leitfähige Struktur in Anwesenheit eines in die Kammer eingespülten Lysemittels, um die an der Mehrzahl von Durchbrüchen zurückgehaltene Mehrzahl von Zellen elektrisch oder thermisch zu lysieren. Ein dritter Prozessabschnitt des Amplifizierens bzw. Vervielfältigens einer im Schritt 904 aus den Zellen herausgelösten Zellsubstanz wie z. B. einer DNA umfasst einen Schritt 906 des erneuten Anlegens der elektrischen Spannung an die elektrisch leitfähige Struktur, um in Anwesenheit eines Reaktionsmittels in der Kammer eine Mehrzahl von aus den Zellen freigesetzten Zellbestandteilen zu vervielfältigen.

Fig. 10 zeigt schematisch eine Aufgliederung des durch den Schritt 902 gekennzeichneten ersten Prozessabschnitts des Verfahrens 900, in dem die Filtration der Analyseprobe erfolgt, in einzelnen Teilsequenzen, gemäß einem Ausführungsbeispiel der vorliegenden Erfindung. Eine Teilsequenz 902A kennzeichnet einen Anfangszustand, in dem die Probenlösung mit den Zielzellen über einen Fluidkanal in den ersten Kammerabschnitt der Kammer eingelassen wurde und dort zur weiteren Aufbereitung vorliegt. In einer Teilsequenz 902B erfolgt eine Zugabe eines Verdünnungs- bzw. Vereinzelungspuffers in die Kammer. In einer Teilsequenz 902C wird die Probenlösung durch den Filter zwischen dem ersten Kammerabschnitt und dem zweiten Kammerabschnitt bewegt bzw. gespült. In einer Teilsequenz 902D werden Inhibitoren und Hintergrund-DNA aus der Probenlösung herausgespült. Eine Teilsequenz 902E kennzeichnet einen Endzustand des ersten Prozessabschnitts, in dem die Zielzellen an der Filtermembran fixiert sind.

Ziel des in Fig. 10 aufgeschlüsselten ersten Prozessabschnittes ist die Fixierung von Zielzellen, beispielsweise von Pathogenen oder zirkulierenden Tumorzellen, auf der Filtermembran und das gleichzeitige Entfernen von PCR-Inhibitoren wie z. B. Hämoglobin und anderen unerwünschten Zellen. Die Größe der Poren der Filtermembran hängt dabei von der spezifischen Anwendung ab. So werden für die Filtration von Bakterien und Pilzen Porengrößen zwischen 0,2 und 1,0 µm benötigt und für die Filtration von zirkulierenden Tumorzellen Porengrößen zwischen 5 µm und 30 µm benötigt.

Für den Fall, dass die Zielzellen Pathogene wie Bakterien oder Pilze sind, werden die eukaryotischen Zellen zunächst lysiert. Das kann beispielsweise mittels chaotrophen Salzen erfolgen. Für den Fall, dass die Zielzellen Tumorzellen sind, braucht keine derartige Probenvorbereitung durchgeführt zu werden. Einer Probenlösung können zusätzlich Reagenzien beigesetzt werden, die die Fließfähigkeit der Probe verbessern oder die Vereinzelung der Zielzellen begünstigen. Eine solche Probenlösung wird dann durch den Filter gespült. Die Porengröße ist dabei so gewählt, dass die Zielzellen in den Poren des Filters stecken bleiben und die anderen Bestandteile - im Falle von Pathogenen Zellreste der lysierten eukaryotischen Zellen, im Falle von Tumorzellen alle gesunden und damit normalgroßen Zellen - durch die Poren hindurch gespült werden. Anschließend werden auf dem Filter anhaftende Bestandteile mit einem Waschpuffer weggespült. Als Resultat dieses Verfahrensschrittes 902 hat man die Zielzellen der Probenlösung auf dem Filter fixiert und die unerwünschten und/oder störenden Substanzen der Probenlösung weggespült bzw. entfernt.

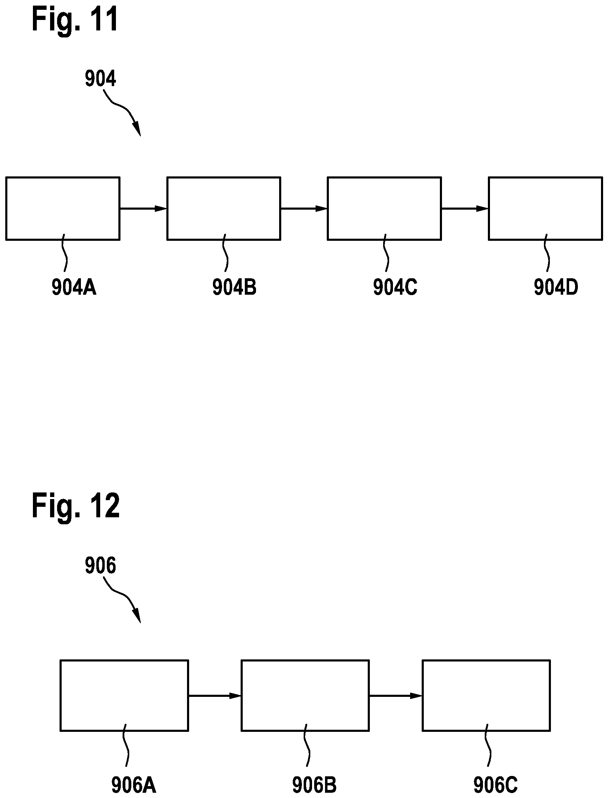

Fig. 11 zeigt schematisch eine Aufgliederung des durch den Schritt 904 gekennzeichneten zweiten Prozessabschnitts des Verfahrens 900, in dem die elektrische und/oder thermische Lyse der Analyseprobe erfolgt, in einzelnen Teilsequenzen, gemäß einem Ausführungsbeispiel der vorliegenden Erfindung. Eine Teilsequenz 904A kennzeichnet einen Anfangszustand, in dem die Zielzellen an dem Filterelement fixiert sind. In einer Teilsequenz 904B wir das Filterelement mit den an ihm fixierten Zielzellen mit einem Lysemedium umspült. Eine thermische und/oder elektrische Lyse der Zellen erfolgt dann in einer Teilsequenz 904C. Schließlich kennzeichnet eine Teilsequenz 904D einen Endzustand des zweiten Prozessabschnitts, in dem die aufgeschlossenen Zellen vorliegen.

Ziel dieses in Fig. 11 gezeigten zweiten Prozessabschnitts ist das Aufschließen der auf dem Filter fixierten und von einem wässrigen Medium umgebenen Zellen. Bei dem Medium, mit dem der Filter, der die fixierten Zielzellen enthält, in der Teilsequenz 904B umspült wird, kann es sich entweder um einen PCR-Reaktionsmix handeln, der alle PCR-Reaktionskomponenten enthält, oder um Wasser, um in der Nähe der Membran vorgelagerte PCR-Reaktionskomponenten aufzunehmen. Die PCR-Reaktionskomponenten können hierbei gefriergetrocknet (lyophilisiert) vorgelagert oder alternativ in Paraffin eingebettet sein. Der für eine PCR benötigte Reaktionsmix stellt sich dann durch Vermengung der Reaktionskomponenten mit Wasser ein, und zwar im Fall der gefriergetrocknet vorgelagerten PCR-Reaktionskomponenten durch Rehydrierung und im Fall der in Paraffin eingebetteten PCR-Reaktionskomponenten durch Aufschmelzen des Paraffins.

In der Teilsequenz 904C werden die Zellen in dem wie oben beschriebenen Medium lysiert. Dies kann thermisch durch Aufheizen der Filtermembran und/oder des umgebenden Mediums auf 80 °C bis 120 °C, bevorzugt auf 90 °C bis 100 °C erfolgen. Bevorzugterweise wird in der Teilsequenz 904C eine sogenannte HotStart-Polymerase eingesetzt. Die thermische Lyse hat dann zusätzlich den Vorteil, dass dabei die Polymerase gleichzeitig aktiviert wird. Die Zielzellen können auch durch Anlegen eines elektrischen Feldes lysiert werden. Dabei kann entweder ein alternierendes elektrisches Wechselfeld von beispielsweise 0,1 x 10^6 V/m bis 10 x 10^6 V/m oder ein konstantes elektrisches Feld oder eine Kombination aus beiden verwendet werden. Nach der Lyse enthält das Medium in der Teilsequenz 904D die zelluläre DNA der lysierten Zellen, Überreste der Zellen und zusätzlich alle Komponenten für eine im dritten Prozessabschnitt durchzuführende PCR.

Fig. 12 zeigt schematisch eine Aufgliederung des durch den Schritt 906 gekennzeichneten dritten Prozessabschnitts des Verfahrens 900, in dem die Vervielfältigung bzw. Amplifikation der aus den Zellen herausgelösten DNA erfolgt, in einzelnen Teilsequenzen, gemäß einem Ausführungsbeispiel der vorliegenden Erfindung. Eine Teilsequenz 906A kennzeichnet einen Anfangszustand, in dem die aufgeschlossenen Zellen vorliegen. In einer Teilsequenz 906B erfolgt eine biochemische Reaktion, hier eine PCR. Schließlich kennzeichnet eine Teilsequenz 906C einen Endzustand des dritten Prozessabschnitts, in dem die vervielfältigten DNA-Abschnitte vorliegen.

Ziel dieses in Fig. 12 gezeigten dritten Prozessabschnitts ist die Amplifikation von bestimmten Abschnitten der zellulären DNA. Die Amplifikation findet dabei in Gegenwart des Filters statt. Das Medium, das die zelluläre DNA der lysierten Zellen enthält, ist vorzugsweise ein PCR-Reaktionsmix. Durch Beaufschlagung des Mediums mit zwischen 50 °C und 95 °C alternierenden Temperaturen wird in der Teilsequenz 906B eine PCR durchgeführt. Eine Identifikation der einzelnen PCR-Produkte erfolgt zum Beispiel in einem anderen Bereich als dem des Filters. Hierfür können prinzipiell zwei Verfahren eingesetzt werden.

In einem ersten Verfahren wird ein Microarray-basierter Nachweis geführt. Hier ist die oben erwähnte PCR beispielsweise eine asymmetrische Multiplex-PCR. Es werden zwischen 1 und 20 Primerpaare eingesetzt und zwischen 20 und 50 PCR-Temperaturzyklen durchgeführt. Die Primer sind mit einem Fluorophor markiert. Somit tragen die PCR-Produkte ein fluoreszentes Label und generieren auf dem Microarray detektierbare Signale. Damit sind qualitative Rückschlüsse auf das Vorhandensein bestimmter DNA-Abschnitte in der Probenlösung möglich. Ein zweites Verfahren wird als Nested-PCR bezeichnet. Hier ist die oben erwähnte PCR eine Multiplex-PCR. Es werden zwischen 1 und 500 Primerpaare eingesetzt, bevorzugte zwischen 1 und 50 Primerpaare und zwischen 10 und 40 PCR-Temperaturzyklen durchgeführt. Die PCR-Produkte der ersten PCR werden verdünnt und auf separate Reaktionskammern verteilt, wobei in jeder Kammer 1 - 4 Primerpaare vorgelagert sind. In diesen Kammern wird eine zweite PCR-Reaktion durchgeführt. Der Anstieg von Signalen dieser Reaktionen wird in Echtzeit detektiert, weshalb eine derartige PCR auch als "Realtime-PCR" bezeichnet werden kann. Damit sind quantitative Rückschlüsse auf das Vorhandensein bestimmter DNA-Abschnitte in der Probenlösung möglich.

Das hierin vorgestellte Konzept der Zellaufbereitung und/oder -diagnose unter Verwendung einer in einer Kammer angeordneten Kombination aus Filterelement und elektrisch leitfähiger Struktur kann für analytische Systeme, insbesondere für mikrofluidische Lab-on-Chip-Systeme, zur Umweltanalytik oder medizinischen Diagnostik verwendet werden.

Die beschriebenen und in den Figuren gezeigten Ausführungsbeispiele sind nur beispielhaft gewählt.

Ferner können die hier vorgestellten Verfahrensschritte wiederholt sowie in einer anderen als in der beschriebenen Reihenfolge ausgeführt werden.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

## Patentansprüche

1. Vorrichtung (100) zum Aufbereiten einer biologischen Probe, wobei die Vorrichtung (100) die folgenden Merkmale aufweist:
ein erstes Substrat (102) mit einer ersten Kavität (106), die einen ersten Kammerabschnitt einer Kammer (110) zum Aufnehmen der biologischen Probe bildet;
ein zweites Substrat (104) mit einer zweiten Kavität (108), die einen zweiten Kammerabschnitt der Kammer (110) zum Aufnehmen der biologischen Probe bildet;
ein Filterelement (112) mit einer Mehrzahl von Durchbrüchen (114), wobei das Filterelement (112) zwischen der ersten Kavität (106) und der zweiten Kavität (108) ausgebildet ist, um bei einem Bewegen der biologischen Probe zwischen der ersten Kavität (106) und der zweiten Kavität (108) durch das Filterelement (112) eine Mehrzahl von organischen Zellen der biologischen Probe auf der Mehrzahl von Durchbrüchen (114) zurückzuhalten; und
eine elektrisch leitfähige Struktur (306), die an dem Filterelement (112) angeordnet und ausgebildet ist, um eine Lyse der organischen Zellen herbeizuführen und/oder definierte Abschnitte der freigelegten DNA zu vervielfältigen und/oder nachzuweisen.

2. Vorrichtung (100) gemäß Anspruch 1, **dadurch gekennzeichnet, dass** das erste Substrat (102) und das zweite Substrat (104) zumindest teilweise aus Silizium gebildet sind.

3. Vorrichtung (100) gemäß einem der vorangegangenen Ansprüche, **dadurch gekennzeichnet, dass** die Vorrichtung (100) einen ersten Fluidkanal (116) zwischen einer von der ersten Kavität (106) abgewandten Hauptseite (304) des ersten Substrats (102) und der ersten Kavität (106) und einen zweiten Fluidkanal (118) zwischen der Hauptseite (304) des ersten Substrats (102) und der zweiten Kavität (108) aufweist, wobei der erste Fluidkanal (116) und der zweite Fluidkanal (118) ausgebildet sind, um die biologische Probe in die Kammer (110) einzulassen und/oder aus der Kammer (110) auszulassen.

4. Vorrichtung (100) gemäß einem der vorangegangenen Ansprüche, **dadurch gekennzeichnet, dass** das zweite Substrat (104) einen die zweite Kavität (108) aufweisenden ersten Substratabschnitt (200) aufweist und einen benachbart zu einem eine Wand der Kammer (110) bildenden Boden (300) der zweiten Kavität (108) angeordneten zweiten Substratabschnitt (202) aufweist, wobei der erste Substratabschnitt (200) aus Silizium gebildet ist und der zweite Substratabschnitt (202) aus Glas gebildet ist.

5. Vorrichtung (100) gemäß einem der vorangegangenen Ansprüche, **dadurch gekennzeichnet, dass** die elektrisch leitfähige Struktur (306) eine erste Leiterbahn (600) zwischen einem ersten Anschlussbereich (604) der elektrisch leitfähigen Struktur (306) und einem zweiten Anschlussbereich (606) der elektrisch leitfähigen Struktur (306) zum Anlegen einer ersten Spannung zwischen dem ersten Anschlussbereich (604) und dem zweiten Anschlussbereich (606) und eine zweite Leiterbahn (602) zwischen einem dritten Anschlussbereich (608) der elektrisch leitfähigen Struktur (306) und einem vierten Anschlussbereich (610) der elektrisch leitfähigen Struktur (306) zum Anlegen einer zweiten Spannung zwischen dem dritten Anschlussbereich (608) und dem vierten Anschlussbereich (610) aufweist, insbesondere wobei die erste Leiterbahn (600) in einem ersten Mäander entlang der Mehrzahl von Durchbrüchen (114) des Filterelements (112) von dem ersten Anschlussbereich (604) zu dem zweiten Anschlussbereich (606) verläuft und die zweite Leiterbahn (602) in einem zu dem ersten Mäander parallelen zweiten Mäander entlang der Mehrzahl von Durchbrüchen (114) des Filterelements (112) von dem dritten Anschlussbereich (608) zu dem vierten Anschlussbereich (610) verläuft.

6. Vorrichtung (100) gemäß Anspruch 5, **dadurch gekennzeichnet, dass** die erste Leiterbahn (600) zumindest einen ersten Leiterbahnabschnitt (616) aufweist, und die zweite Leiterbahn (602) zumindest einen zweiten Leiterbahnabschnitt (618) aufweist, wobei der erste Leiterbahnabschnitt (616) sich zu einem Rand (612) eines Durchbruchs (114) erstreckt und der zweite Leiterbahnabschnitt (618) sich zu einem dem Rand (612) gegenüberliegenden weiteren Rand (614) des Durchbruchs (114) erstreckt.

7. Vorrichtung (100) gemäß einem der Ansprüche 3 bis 6, **dadurch gekennzeichnet, dass** für eine elektrische Kontaktierung der elektrisch leitfähigen Struktur (306) das erste Substrat (102) einen ersten Durchtritt (308) zwischen der Hauptseite (304) des ersten Substrats (102) und der elektrisch leitfähigen Struktur (306) aufweist und/oder das zweite Substrat (104) einen zweiten Durchtritt (310) zwischen einer von der zweiten Kavität (108) abgewandten Hauptseite (312) des zweiten Substrats (104) und der elektrisch leitfähigen Struktur (306) aufweist.

8. Vorrichtung (100) gemäß einem der Ansprüche 4 bis 7, **dadurch gekennzeichnet, dass** die Vorrichtung (100) eine weitere leitfähige Struktur (400) aufweist, wobei die weitere leitfähige Struktur (400) an dem Boden (300) der zweiten Kavität (108) angeordnet ist.

9. Vorrichtung (100) gemäß einem der Ansprüche 3 bis 8, **dadurch gekennzeichnet, dass** die Vorrichtung (100) einen Detektionsbereich (500) zum Aufnehmen eines bei der Lyse der organischen Zellen gewonnenen Lysats aufweist, wobei der Detektionsbereich (500) benachbart zu dem ersten Fluidkanal (116) und/oder dem zweiten Fluidkanal (118) an der Hauptseite (304) des ersten Substrats (102) angeordnet ist.

10. Analysesystem (700) zum Analysieren einer biologischen Probe, wobei das Analysesystem (700) die folgenden Merkmale aufweist:
eine Vorrichtung (100) zum Aufbereiten der biologischen Probe organischer Zellen gemäß Anspruch 9; und
eine Detektionseinheit (702) zum Detektieren eines in der Vorrichtung (100) aus den organischen Zellen herausgelösten vorbestimmten Zellbestandteils der biologischen Probe, wobei die Detektionseinheit (702) fluidisch mit der Vorrichtung (100) gekoppelt ist.

11. Analysesystem (700) gemäß Anspruch 10, **dadurch gekennzeichnet, dass** die Detektionseinheit (702) einen Schichtaufbau aus einem ein Kanalsystem (710) aufweisenden ersten Polymersubstrat (704), einem eine Ausnehmung (712) aufweisenden zweiten Polymersubstrat (708) und einer zwischen dem ersten Polymersubstrat (704) und dem zweiten Polymersubstrat (708) angeordneten, ein mikrofluidisches Netzwerk aufweisenden, Mikrofluidschicht (706) sowie eine mit dem Kanalsystem (710) gekoppelte Analysekavität (714) zur Durchführung eines Analyseverfahrens an dem in der Vorrichtung (100) gewonnen Lysat aufweist, wobei die Vorrichtung (100) derart in der Ausnehmung (712) und angrenzend an die Mikrofluidschicht (706) angeordnet ist, dass der Detektionsbereich (500) in der Analysekavität (714) liegt und der erste Fluidkanal (116) und/oder der zweite Fluidkanal (118) fluidisch mit dem Kanalsystem (710) gekoppelt ist.

12. Verfahren (900) zum Aufbereiten einer biologischen Probe, wobei das Verfahren (900) den folgenden Schritt aufweist:
Bewegen (902) der biologischen Probe zwischen einer in einem ersten Substrat (102) angelegten, einen ersten Kammerabschnitt einer Kammer (110) zum Aufnehmen der biologischen Probe bildenden, ersten Kavität (106) und einer in einem zweiten Substrat (104) angelegten, einen zweiten Kammerabschnitt der Kammer (110) bildenden, zweiten Kavität (108) durch ein zwischen der ersten Kavität (106) und der zweiten Kavität (108) ausgebildetes Filterelement (112) mit einer Mehrzahl von Durchbrüchen (114) und eine an dem Filterelement (112) angeordnete elektrisch leitfähige Struktur (306), um eine Mehrzahl von organischen Zellen der biologischen Probe auf der Mehrzahl von Durchbrüchen (114) zurückzuhalten; und
Anlegen (904) zumindest einer elektrischen Spannung an die elektrisch leitfähige Struktur (306) in Anwesenheit eines in die Kammer (110) eingespülten Lysemittels, um die an der Mehrzahl von Durchbrüchen (114) zurückgehaltene Mehrzahl von organischen Zellen zu lysieren.

13. Verfahren (900) gemäß Anspruch 12, **dadurch gekennzeichnet, dass** in dem Schritt des Anlegens (904) die elektrische Spannung über eine einen ersten Anschlussbereich (604) der elektrisch leitfähigen Struktur (306) mit einem zweiten Anschlussbereich (606) der elektrisch leitfähigen Struktur (306) verbindende erste Leiterbahn (600) angelegt wird, um die an der Mehrzahl von Durchbrüchen (114) zurückgehaltene Mehrzahl von organischen Zellen basierend auf einer Joulschen Erwärmung des Filterelements (112) thermisch zu lysieren.

14. Verfahren (900) gemäß Anspruch 12 oder 13, **dadurch gekennzeichnet, dass** in dem Schritt des Anlegens (904) eine weitere elektrische Spannung über eine einen dritten Anschlussbereich (608) der elektrisch leitfähigen Struktur (306) mit einem vierten Anschlussbereich (610) der elektrisch leitfähigen Struktur (306) verbindende zweite Leiterbahn (602) angelegt wird, um die an der Mehrzahl von Durchbrüchen (114) zurückgehaltene Mehrzahl von organischen Zellen basierend auf einer Erzeugung eines elektrischen Feldes zwischen der ersten Leiterbahn (600) und der zweiten Leiterbahn (602) elektrisch zu lysieren.

15. Verfahren (900) gemäß einem der Ansprüche 12 bis 14, **dadurch gekennzeichnet, dass** das Verfahren (900) einen Schritt des erneuten Anlegens (906) der elektrischen Spannung an die elektrisch leitfähige Struktur (306) aufweist, um in Anwesenheit eines Reaktionsmittels in der Kammer (110) eine Mehrzahl von bei dem Lysieren aus der Mehrzahl von organischen Zellen freigesetzten Zellbestandteilen zu vervielfältigen.

## Claims

1. Apparatus (100) for processing a biological sample, the apparatus (100) comprising the following features:
a first substrate (102) with a first cavity (106) which forms a first chamber portion of a chamber (110) for receiving the biological sample;
a second substrate (104) with a second cavity (108) which forms a second chamber portion of the chamber (110) for receiving the biological sample;
a filter element (112) with a plurality of perforations (114), the filter element (112) being embodied between the first cavity (106) and the second cavity (108) in order to hold back a plurality of organic cells of the biological sample at the plurality of perforations (114) when moving the biological sample between the first cavity (106) and the second cavity (108) through the filter element (112); and
an electrically conductive structure (306) which is arranged at the filter element (112) and embodied to bring about lysis of the organic cells and/or to duplicate and/or detect defined sections of the exposed DNA.

2. Apparatus (100) according to Claim 1, **characterized in that** the first substrate (102) and the second substrate (104) are at least partly formed from silicon.

3. Apparatus (100) according to one of the preceding claims, **characterized in that** the apparatus (100) comprises a first fluid channel (116) between a main side (304) of the first substrate (102) facing away from the first cavity (106) and the first cavity (106) and a second fluid channel (118) between the main side (304) of the first substrate (102) and the second cavity (108), the first fluid channel (116) and the second fluid channel (118) being embodied to admit the biological sample into the chamber (110) and/or release it from the chamber (110).

4. Apparatus (100) according to one of the preceding claims, **characterized in that** the second substrate (104) comprises a first substrate portion (200) comprising the second cavity (108) and a second substrate portion (202) arranged adjacent to a base (300) of the second cavity (108) forming a wall of the chamber (110), the first substrate portion (200) being made of silicon and the second substrate portion (202) being made of glass.

5. Apparatus (100) according to one of the preceding claims, **characterized in that** the electrically conductive structure (306) comprises a first conductor track (600) between a first connection region (604) of the electrically conductive structure (306) and a second connection region (606) of the electrically conductive structure (306) for applying a first voltage between the first connection region (604) and the second connection region (606), and a second conductor track (602) between a third connection region (608) of the electrically conductive structure (306) and a fourth connection region (610) of the electrically conductive structure (306) for applying a second voltage between the third connection region (608) and the fourth connection region (610), in particular with the first conductor track (600) extending in a first meandering structure along the plurality of perforations (114) of the filter element (112) from the first connection region (604) to the second connection region (606) and the second conductor track (602) extending in a second meandering structure parallel to the first meandering structure along the plurality of perforations (114) of the filter element (112) from the third connection region (608) to the fourth connection region (610).

6. Apparatus (100) according to Claim 5, **characterized in that** the first conductor track (600) has at least one first conductor track portion (616) and the second conductor track (602) has at least one second conductor track portion (618), the first conductor track portion (616) extending to an edge (612) of a perforation (114) and the second conductor track portion (618) extending to a further edge (614) of the perforation (114) lying opposite the edge (612).

7. Apparatus (100) according to one of Claims 3 to 6, **characterized in that,** for electrically contacting the electrically conductive structure (306), the first substrate (102) has a first feedthrough (308) between the main side (304) of the first substrate (102) and the electrically conductive structure (306) and/or the second substrate (104) has a second feedthrough (310) between a main side (312) of the second substrate (104) facing away from the second cavity (108) and the electrically conductive structure (306).

8. Apparatus (100) according to one of Claims 4 to 7, **characterized in that** the apparatus (100) comprises a further conductive structure (400), the further conductive structure (400) being arranged at the base (300) of the second cavity (108).

9. Apparatus (100) according to one of Claims 3 to 8, **characterized in that** the apparatus (100) comprises a detection region (500) for receiving a lysate obtained during the lysis of the organic cells, the detection region (500) being arranged adjacent to the first fluid channel (116) and/or the second fluid channel (118) at the main side (304) of the first substrate (102).

10. Analysis system (700) for analyzing a biological sample, the analysis system (700) comprising the following features:
an apparatus (100) for processing the biological sample of organic cells according to Claim 9; and
a detection unit (702) for detecting a predetermined cell constituent of the biological sample extracted from the organic cells in the apparatus (100), the detection unit (702) being fluidically coupled to the apparatus (100).

11. Analysis system (700) according to Claim 10, **characterized in that** the detection unit (702) comprises a layer structure made of a first polymer substrate (704) comprising a channel system (710), a second polymer substrate (708) comprising a recess (712) and a microfluidic layer (706) arranged between the first polymer substrate (704) and the second polymer substrate (708) and comprising a microfluidic network, and also an analysis cavity (714), coupled to the channel system (710), for performing an analysis method on the lysate obtained in the apparatus (100), the apparatus (100) being arranged in the recess (712) and adjacent to the microfluidic layer (706) in such a way that the detection region (500) lies in the analysis cavity (714) and the first fluid channel (116) and/or the second fluid channel (118) is fluidically coupled to the channel system (710).

12. Method (900) for processing a biological sample, the method (900) comprising the following step:
moving (902) the biological sample between a first cavity (106), which is laid out in a first substrate (102) and forms a first chamber portion of a chamber (110) for receiving the biological sample, and a second cavity (108), which is laid out in a second substrate (104) and forms a second chamber portion of the chamber (110), through a filter element (112), which comprises a plurality of perforations (114), embodied between the first cavity (106) and the second cavity (108), and an electrically conductive structure (306) arranged at the filter element (112) in order to hold back a plurality of organic cells of the biological sample at the plurality of perforations (114); and
applying (904) at least one electric voltage to the electrically conductive structure (306) in the presence of a lysing agent flushed into the chamber (110) in order to lyse the plurality of organic cells held back at the plurality of perforations (114).

13. Method (900) according to Claim 12, **characterized in that** the electric voltage is applied in the applying (904) step via a first conductor track (600) connecting a first connection region (604) of the electrically conductive structure (306) with a second connection region (606) of the electrically conductive structure (306) in order to thermally lyse the plurality of organic cells held back at the plurality of perforations (114) on the basis of Joule heating of the filter element (112) .

14. Method - (900) according to Claim 12 or 13, **characterized in that** a further electric voltage is applied in the applying (904) step via a second conductor track (602) connecting a third connection region (608) of the electrically conductive structure (306) to a fourth connection region (610) of the electrically conductive structure (306) in order to electrically lyse the plurality of organic cells held back at the plurality of perforations (114) on the basis of generating an electric field between the first conductor track (600) and the second conductor track (602).

15. Method (900) according to one of Claims 12 to 14, **characterized in that** the method (900) comprises a step of renewed application (906) of the electric voltage to the electrically conductive structure (306) in order to duplicate a plurality of cell constituents released from the plurality of organic cells during lysis if a reaction agent is present in the chamber (110).

## Revendications

1. Dispositif (100) pour préparer un échantillon biologique, le dispositif (100) possédant les caractéristiques suivantes :
un premier substrat (102) pourvu d'une première cavité (106), qui forme une première portion de chambre d'une chambre (110) destinée à accueillir l'échantillon biologique ;
un deuxième substrat (104) pourvu d'une deuxième cavité (108), qui forme une deuxième portion de chambre de la chambre (110) destinée à accueillir l'échantillon biologique ;
un élément filtrant (112) comprenant une pluralité de traversées (114), l'élément filtrant (112) étant formé entre la première cavité (106) et la deuxième cavité (108) afin de retenir une pluralité de cellules organiques de l'échantillon biologique sur la pluralité de traversées (114) lors d'un déplacement de l'échantillon biologique entre la première cavité (106) et la deuxième cavité (108) à travers l'élément filtrant (112) ; et
une structure électriquement conductrice (306), qui est disposée au niveau de l'élément filtrant (112) et configurée pour causer une lyse des cellules organiques et/ou reproduire et/ou déceler des portions définies de l'ADN libéré.

2. Dispositif (100) selon la revendication 1, **caractérisé en ce que** le premier substrat (102) et le deuxième substrat (104) sont au moins partiellement constitués de silicium.

3. Dispositif (100) selon l'une des revendications précédentes, **caractérisé en ce que** le dispositif (100) possède un premier canal à fluide (116) entre un côté principal (304) du premier substrat (102) à l'opposé de la première cavité (106) et la première cavité (106) et un deuxième canal à fluide (118) entre le côté principal (304) du premier substrat (102) et la deuxième cavité (108), le premier canal à fluide (116) et le deuxième canal à fluide (118) étant configurés pour laisser entrer l'échantillon biologique dans la chambre (110) et/ou le laisser sortir de la chambre (110).

4. Dispositif (100) selon l'une des revendications précédentes, **caractérisé en ce que** le deuxième substrat (104) possède une première portion de substrat (200) qui présente la deuxième cavité (108) et une deuxième portion de substrat (202) disposée adjacente à un fond (300) de la deuxième cavité (108) qui forme une paroi de la chambre (110), la première portion de substrat (200) étant constituée de silicium et la deuxième portion de substrat (202) étant constituée de verre.

5. Dispositif (100) selon l'une des revendications précédentes, **caractérisé en ce que** la structure électriquement conductrice (306) possède une première piste conductrice (600) entre une première zone de raccordement (604) de la structure électriquement conductrice (306) et une deuxième zone de raccordement (606) de la structure électriquement conductrice (306) destinée à appliquer une première tension entre la première zone de raccordement (604) et la deuxième zone de raccordement (606), et une deuxième piste conductrice (602) entre une troisième zone de raccordement (608) de la structure électriquement conductrice (306) et une quatrième zone de raccordement (610) de la structure électriquement conductrice (306) destinée à appliquer une deuxième tension entre la troisième zone de raccordement (608) et la quatrième zone de raccordement (610), la première piste conductrice (600) suivant notamment un tracé dans un premier méandre le long de la pluralité de traversées (114) de l'élément filtrant (112) de la première zone de raccordement (604) à la deuxième zone de raccordement (606), et la deuxième piste conductrice (602) suivant un tracé dans un deuxième méandre, parallèle au premier méandre, le long de la pluralité de traversées (114) de l'élément filtrant (112) de la troisième zone de raccordement (608) à la quatrième zone de raccordement (610).

6. Dispositif (100) selon la revendication 5, **caractérisé en ce que** la première piste conductrice (600) possède au moins une première portion de piste conductrice (616) et la deuxième piste conductrice (602) au moins une deuxième portion de piste conductrice (618), la première portion de piste conductrice (616) s'étendant vers un bord (612) d'une traversée (114) et la deuxième portion de piste conductrice (618) s'étendant vers un autre bord (614) de la traversée (114), qui se trouve à l'opposé du bord (612).

7. Dispositif (100) selon l'une des revendications 3 à 6, **caractérisé en ce que** pour une mise en contact électrique de la structure électriquement conductrice (306), le premier substrat (102) possède un premier passage (308) entre le côté principal (304) du premier substrat (102) et la structure électriquement conductrice (306) et/ou le deuxième substrat (104) possède un deuxième passage (310) entre un côté principal (312) du deuxième substrat (104) à l'opposé de la deuxième cavité (108) et la structure électriquement conductrice (306).

8. Dispositif (100) selon l'une des revendications 4 à 7, **caractérisé en ce que** le dispositif (100) possède une structure conductrice supplémentaire (400), la structure conductrice supplémentaire (400) étant disposée au niveau du fond (300) de la deuxième cavité (108).

9. Dispositif (100) selon l'une des revendications 3 à 8, **caractérisé en ce que** le dispositif (100) possède une zone de détection (500) destinée à accueillir un lysat obtenu lors de la lyse des cellules organiques, la zone de détection (500) étant disposée adjacente au premier canal à fluide (116) et/ou au deuxième canal à fluide (118) au niveau du côté principal (304) du premier substrat (102).

10. Système d'analyse (700) destiné à analyser un échantillon biologique, le système d'analyse (700) possédant les caractéristiques suivantes :
un dispositif (100) destiné à préparer l'échantillon biologique de cellules organiques selon la revendication 9 ; et
une unité de détection (702) destinée à détecter une composante cellulaire prédéterminée de l'échantillon biologique qui se détache des cellules organiques dans le dispositif (100), l'unité de détection (702) étant couplée fluidiquement avec le dispositif (100).

11. Système d'analyse (700) selon la revendication 10, **caractérisé en ce que** l'unité de détection (702) possède une structure stratifiée composée d'un premier substrat en polymère (704) qui possède un système de canal (710), d'un deuxième substrat en polymère (708) qui possède un évidement (712) et d'une couche microfluidique (706) disposée entre le premier substrat en polymère (704) et le deuxième substrat en polymère (708) et qui possède un réseau microfluidique, ainsi qu'une cavité d'analyse (714) couplée au système de canal (710) et destinée à mettre en œuvre un procédé d'analyse sur un lysat obtenu dans le dispositif (100), le dispositif (100) étant disposé dans l'évidement (712) et adjacent à la couche microfluidique (706) de telle sorte que la zone de détection (500) se trouve dans la cavité d'analyse (714) et le premier canal à fluide (116) et/ou le deuxième canal à fluide (118) est couplé fluidiquement avec le système de canal (710).

12. Procédé (900) pour préparer un échantillon biologique, le procédé (900) comprenant l'étape suivante :
déplacement (902) de l'échantillon biologique entre une première cavité (106), créée dans un premier substrat (102) et formant une première portion de chambre d'une chambre (110) destinée à accueillir l'échantillon biologique, et une deuxième cavité (108), créée dans un deuxième substrat (104) et formant une deuxième portion de chambre de la chambre (110), à travers un élément filtrant (112) formé entre la première cavité (106) et la deuxième cavité (108) et comprenant une pluralité de traversées (114) ainsi qu'une structure électriquement conductrice (306) disposée au niveau de l'élément filtrant (112), afin de retenir une pluralité de cellules organiques de l'échantillon biologique sur la pluralité de traversées (114) ; et
application (904) d'au moins une tension électrique à la structure électriquement conductrice (306) en présence d'un milieu de lyse injecté dans la chambre (110) afin d'effectuer une lyse de la pluralité de cellules organiques retenues au niveau de la pluralité de traversées (114).

13. Procédé (900) selon la revendication 12, **caractérisé en ce que** dans l'étape d'application (904), la tension électrique est appliquée par le biais d'une première piste conductrice (600) qui relie une première zone de raccordement (604) de la structure électriquement conductrice (306) à une deuxième zone de raccordement (606) de la structure électriquement conductrice (306) afin de réaliser une thermolyse de la pluralité de cellules organiques retenues au niveau de la pluralité de traversées (114) en se basant sur un échauffement (effet joule) de l'élément filtrant (112).

14. Procédé (900) selon la revendication 12 ou 13, **caractérisé en ce que** dans l'étape d'application (904), une tension électrique supplémentaire est appliquée par le biais d'une deuxième piste conductrice (602) qui relie une troisième zone de raccordement (608) de la structure électriquement conductrice (306) à une quatrième zone de raccordement (610) de la structure électriquement conductrice (306) afin de réaliser une électrolyse de la pluralité de cellules organiques retenues au niveau de la pluralité de traversées (114) en se basant sur une génération d'un champ électrique entre la première piste conductrice (600) et la deuxième piste conductrice (602).

15. Procédé (900) selon l'une des revendications 12 à 14, **caractérisé en ce que** le procédé (900) comprend une étape de réapplication (906) de la tension électrique à la structure électriquement conductrice (306) afin de, en présence d'un réactif dans la chambre (110), reproduire une pluralité de composantes cellulaires libérées de la pluralité de cellules organiques lors de la lyse.
